(19) Europäisches Patentamt
European Patent Office
Office européen des brevets

(11) Veröffentlichungsnummer: **0 055 444**
**B1**

# (12) EUROPÄISCHE PATENTSCHRIFT

(45) Veröffentlichungstag der Patentschrift: **20.03.85**

(51) Int. Cl.⁴: **C 07 D 487/04**

(21) Anmeldenummer: **81110635.0**

(22) Anmeldetag: **21.12.81**

(54) **Trisubstituierte Pyrimido(5,4-d)pyrimidine, ihre Herstellung und diese enthaltende Arzneimittel.**

(30) Priorität: **27.12.80 DE 3049207**

(43) Veröffentlichungstag der Anmeldung: **07.07.82 Patentblatt 82/27**

(45) Bekanntmachung des Hinweises auf die Patenterteilung: **20.03.85 Patentblatt 85/12**

(84) Benannte Vertragsstaaten: **AT BE CH DE FR IT LI LU NL SE**

(56) Entgegenhaltungen: **EP - A - 0 023 559** **FR - A - 1 292 943**

(73) Patentinhaber: **Dr. Karl Thomae GmbH, Postfach 1755, D-7950 Biberach (Riss) (DE)**

(72) Erfinder: **Roch, Josef, Dr. Dipl.-Chem., Stecherweg 19, D-7950 Biberach 1 (DE)**
Erfinder: **Müller, Erich, Dr. Dipl.-Chem., Talfeldstrasse 34, D-7950 Biberach 1 (DE)**
Erfinder: **Narr, Berthold, Dr. Dipl.-Chem., Obere Au 5, D-7950 Biberach 1 (DE)**
Erfinder: **Nickl, Josef, Dr. Dipl.-Chem., Silcherstrasse 8, D-7950 Biberach 1 (DE)**
Erfinder: **Haarmann, Walter, Dr., Schlierholzweg 27, D-7950 Biberach 1 (DE)**
Erfinder: **Weisenberger, Johannes M., Dr. Dipl.-Chem., Haydnweg 5, D-7950 Biberach 1 (DE)**

**EP 0 055 444 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Aus der FR-A-1 292 943 sind bereits Pyrimido[5,4-d]pyrimidine bekannt, welche Wirkungen auf den Kreislauf und krampflösende Eigenschaften aufweisen.

Es wurde nun gefunden, dass die beanspruchten trisubstituierte Pyrimido[5,4-d]pyrimidine der allgemeinen Formel (I)

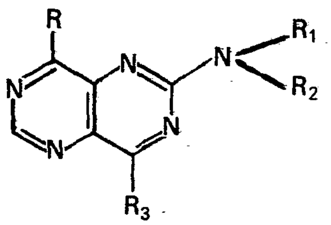

(I)

deren Säureadditionssalze, insbesondere deren physiologisch verträgliche Säureadditionssalze mit anorganischen oder organischen Säuren, wertvolle pharmakologische Eigenschaften, nämlich insbesondere antithrombotische Wirkungen aufweisen.

Gegenstand der Erfindung sind daher die neuen trisubstituierten Pyrimido[5,4-d]pyrimidine der allgemeinen Formel (I), deren Säureadditionssalze, insbesondere deren physiologisch verträgliche Säureadditionssalze mit anorganischen oder organischen Säuren und Verfahren zu ihrer Herstellung sowie diese Verbindungen enthaltende Arzneimittel.

In der obigen allgemeinen Formel (I) bedeutet

$R_1$ eine gegebenenfalls ab Kohlenstoffatom 2 mit bis zu 5 Hydroxygruppen substituierte geradkettige oder verzweigte Alkylgruppe mit 2 bis 8 Kohlenstoffatomen, eine durch eine Alkoxy-, Amino-, Alkylamino-, Dialkylamino- oder Morpholinogruppe in 2-, 3- oder 4-Stellung substituierte Alkylgruppe mit 2 bis 4 Kohlenstoffatomen, wobei die vorstehend erwähnten Alkylsubstituenten jeweils 1 bis 3 Kohlenstoffatome enthalten können, eine Methyl- oder Pyridylmethylgruppe,

$R_2$ ein Wasserstoffatom, eine Alkylgruppe mit 1 bis 3 Kohlenstoffatomen oder eine durch eine Hydroxygruppe oder eine Alkoxygruppe mit 1 bis 3 Kohlenstoffatomen in 2- oder 3-Stellung substituierte Alkylgruppe mit 2 oder 3 Kohlenstoffatomen oder

$R_1$ und $R_2$ zusammen mit dem dazwischenliegenden Stickstoffatom eine Imidazolyl-, Morpholino-, Thiomorpholino-, 1-Oxidothiomorpholino- oder Piperidinogruppe, wobei die Piperidinogruppe durch eine Hydroxygruppe, eine Alkoxygruppe mit 1 bis 3 Kohlenstoffatomen, eine Hydroxyalkylgruppe mit 1 bis 3 Kohlenstoffatomen, eine Alkoxycarbonylgruppe mit insgesamt 2 bis 4 Kohlenstoffatomen, eine Aminogruppe, eine Alkanoylaminogruppe mit 1 bis 3 Kohlenstoffatomen im Alkanoylteil, eine Aminocarbonyl-, Alkylamino- oder Dialkylaminogruppe mit jeweils 1 bis 3 Kohlenstoffatomen im Alkylteil substituiert sein kann,

$R_3$ eine gegebenenfalls durch eine oder zwei Alkylgruppen mit jeweils 1 bis 3 Kohlenstoffatome substituierte Morpholino-, Thiomorpholino-, 1-Oxido-thiomorpholino- oder 1,1-Dioxidothiomorpholinogruppe und

R eine Gruppe der Formel

$-X-R_5$ oder

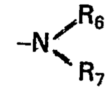, wobei

X ein Sauerstoff- oder Schwefelatom,

$R_5$ eine gegebenenfalls durch eine Phenylgruppe oder eine in 2-, 3- oder 4-Stellung durch eine Hydroxygruppe, eine Dialkylamino- oder Alkoxygruppe mit jeweils 1 bis 3 Kohlenstoffatomen im Alkylteil oder eine Alkoxycarbonylgruppe mit insgesamt 2 bis 4 Kohlenstoffatomen substituierte Alkylgruppe mit 1 bis 4 Kohlenstoffatomen oder, wenn X ein Schwefelatom darstellt, auch eine Phenylgruppe, wobei die vorstehend erwähnten Phenylkerne jeweils durch eine Methylendioxygruppe monosubstituiert oder durch Alkylgruppen mit 1 bis 3 Kohlenstoffatomen, Alkoxygruppen mit 1 bis 3 Kohlenstoffatomen, Alkylthiogruppen mit 1 bis 3 Kohlenstoffatomen, Trifluormethylgruppen und/oder Halogenatome mono- oder disubstituiert sein können, eine Alkylgruppe mit 5 bis 8 Kohlenstoffatomen, eine Cycloalkylgruppe mit 5 bis 7 Kohlenstoffatomen, eine Furfuryl-, Indanyl- oder Naphthylmethylgruppe,

$R_6$ ein Wasserstoffatom, eine Alkylgruppe mit 1 bis 4 Kohlenstoffatomen oder eine durch eine Hydroxygruppe oder Alkoxygruppe mit 1 bis 3 Kohlenstoffatomen in 2-, 3- oder 4-Stellung substituierte Alkylgruppe mit 2 bis 4 Kohlenstoffatomen und

$R_7$ ein Wasserstoffatom, eine gegebenenfalls durch eine Phenylgruppe substituierte Alkylgruppe mit 1 bis 4 Kohlenstoffatomen oder eine Phenylgruppe, wobei die vorstehend erwähnten Phenylkerne jeweils durch eine Methylendioxygruppe monosubstituiert oder durch Alkylgruppen mit 1 bis 3 Kohlenstoffatomen, Alkoxygruppen mit 1 bis 3 Kohlenstoffatomen, Alkylthiogruppen mit 1 bis 3 Kohlenstoffatomen, Trifluormethylgruppen und/oder Halogenatome mono- oder disubstituiert sein können, eine Alkylgruppe mit 5 bis 8 Kohlenstoffatomen, eine Cycloalkylgruppe mit 5 bis 7 Kohlenstoffatomen, eine ab Kohlenstoffatom 2 durch eine Hydroxy- oder Alkoxygruppe mit 1 bis 3 Kohlenstoffatomen substituierte Alkylgruppe mit 2 bis 8 Kohlenstoffatomen, eine Cyclohexylmethyl-, Furfuryl- oder Pyridylmethylgruppe oder

$R_6$ und $R_7$ zusammen mit dem dazwischen liegenden Stickstoffatom eine gegebenenfalls durch eine oder zwei Alkylgruppen mit jeweils 1 bis 3 Kohlenstoffatomen substituierte Alkyleniminogruppe mit 4 bis 7 Kohlenstoffatomen oder eine Tetrahydropyridinogruppe.

Für die bei der Definition der Reste R und $R_1$ bis

$R_7$ eingangs erwähnten Bedeutungen kommt beispielsweise

für $R_1$ die Bedeutung der Methyl-, Äthyl-, Propyl-, Isopropyl-, Butyl-, Isobutyl-, Pentyl-, Isopentyl-, Neopentyl-, Hexyl-, Heptyl-, Octyl-, 2-Aminoäthyl-, 3-Amino-propyl-, 4-Aminobutyl-, 2-Methylaminoäthyl-, 2-Propylaminoäthyl-, 2-Dimethylaminoäthyl-, 3-Diäthylaminopropyl-, 4-Methyläthylaminobutyl-, 2-Morpholinoäthyl-, 2-Hydroxyäthyl-, 3-Hydroxypropyl-, 4-Hydroxybutyl-, 5-Hydroxypentyl-, 6-Hydroxyhexyl-, 7-Hydroxyheptyl-, 8-Hydroxyoctyl-, 2-Hydroxypropyl-, 2,3-Dihydroxypropyl-, 3-Hydroxy-2,2-dimethyl-propyl-, 2,3-Dihydroxybutyl-, 2,4-Dihydroxybutyl-, 2,3,4-Trihydroxybutyl-, 2,3,4,5-Tetrahydroxypentyl-, 2,3,4,5,6-Pentahydroxyhexyl-, 1,5-Dimethyl-5-hydroxyhexyl-, 2-Methoxyäthyl-, 3-Äthoxypropyl-, 2-Propoxyäthyl-, 4-Methoxy-butyl- oder Pyridylmethylgruppe,

für $R_2$ die des Wasserstoffatoms, der Methyl-, Äthyl-, Propyl-, Isopropyl-, 2-Hydroxyäthyl-, 2-Hydroxypropyl-, 3-Hydroxypropyl-, 2-Methoxyäthyl-, 2-Isopropoxyäthyl-, 3-Methoxypropyl- oder 2-Methoxypropylgruppe,

für $R_1$ und $R_2$ zusammen mit dem dazwischen liegenden Stickstoffatom die der Imidazolyl-, Morpholino-, Thiomorpholino-, 1-Oxidothiomorpholino-, Piperidino-, Methylpiperidino-, Äthylpiperidino-, Dimethylpiperidino-, Hydroxypiperidino-, Methoxypiperidino-, Äthoxypiperidino-, Hydroxymethylpiperidino-, 2-Hydroxyäthylpiperidino-, Methoxycarbonylpiperidino-, Äthoxycarbonylpiperidino-, Dimethylaminopiperidino-, Acetylaminopiperidino-, Propionylaminopiperidino- oder Aminocarbonylpiperidinogruppe,

für $R_3$ die der Morpholino-, Methylmorpholino-, Propylmorpholino-, Dimethylmorpholino-, Äthylmethylmorpholino-, Thiomorpholino-, Äthylthiomorpholino-, 1-Oxidothiomorpholino-, 1,1-Dioxidothiomorpholino- oder Methyl-1,1-dioxidothiomorpholinogruppe und

für R die der Methoxy-, Äthoxy-, Propoxy-, Isopropoxy-, Butoxy-, Isobutoxy-, Pentoxy-, Isopentoxy-, Hexoxy-, Heptoxy-, Octyloxy-, Methylthio-, Äthylthio-, Propylthio-, Isopropylthio-, Butylthio-, Isobutylthio-, Pentylthio-, Isopentylthio-, Neopentylthio-, Hexylthio-, Heptylthio-, Octylthio-, Cyclopentoxy-, Cyclohexoxy-, Cycloheptoxy-, Cyclopentylthio-, Cyclohexylthio-, Cycloheptylthio-, 2-Hydroxyäthoxy-, 3-Hydroxypropoxy-, 4-Hydroxybutoxy-, 2-Methoxyäthoxy-, 3-Äthoxypropoxy-, 4-Methoxybutoxy-, Benzyloxy-, Methylbenzyloxy-, Isopropylbenzyloxy-, Fluorbenzyloxy-, Chlorbenzyloxy-, Dichlorbenzyloxy-, Brombenzyloxy-, Methoxybenzyloxy-, Dimethoxy-benzyloxy-, Methylendioxybenzyloxy-, Äthoxybenzyloxy-, Methylthiobenzyloxy-, Trifluormethylbenzyloxy-, 2-Phenyläthoxy-, 3-Phenylpropoxy-, 4-Phenylbutoxy-, 2-Dimethylaminoäthoxy-, 3-Diäthylamino-propoxy-, Methoxycarbonylmethoxy-, 2-Äthoxycarbonyläthoxy-, 2-Hydroxyäthylthio-, 4-Hydroxybutylthio-, 2-Methoxyäthylthio-, 3-Methoxypropylthio-, 4-Methoxybutylthio-, Benzylthio-, Methylbenzylthio-, Äthylbenzylthio-, Fluorbenzylthio-, Chlorbenzylthio-, Brombenzylthio-, Dichlorbenzylthio-, Methoxybenzylthio-, Isopropoxybenzylthio-, Dimethoxybenzylthio-, Methylendioxybenzylthio-, Trifluormethylbenzylthio-, Methylthiobenzylthio-, 2-Phenyläthylthio-, 3-Phenylpropylthio-, 4-Phenylbutylthio-, Äthoxycarbonylmethyl thio-, 2-Diäthylaminoäthylthio-, Furfuryloxy-, Furfurylthio-, Naphthylmethylthio-, Naphthylmethoxy-, Indanylthio-, Indanyloxy-, Phenylthio-, Methylphenylthio-, Propylphenylthio-, Hydroxyphenylthio-, Methoxyphenylthio-, Fluorphenylthio-, Chlorphenylthio-, Dichlorphenylthio-, Bromphenylthio-, Dimethoxyphenylthio-, Methylendioxyphenylthio-, Methylthiophenylthio-, Amino-, Methylamino-, Äthylamino-, Propylamino-, Isopropylamino-, Butylamino-, Isobutylamino-, Pentylamino-, Isopentylamino-, Neopentylamino-, Hexylamino-, Heptylamino-, Octylamino-, Cyclohexylamino-, 2-Hydroxyäthylamino-, 3-Hydroxypropylamino-, 4-Hydroxybutylamino-, 5-Hydroxypentylamino-, 8-Hydroxyoctylamino-, 1,5-Dimethyl-5-hydroxy-hexylamino-, 2-Methoxyäthylamino-, 3-Methoxypropylamino-, 3-Äthoxypropylamino-, Benzylamino-, 1-Phenyläthylamino-, 2-Phenyläthylamino-, 3-Phenylpropylamino-, 4-Phenylbutylamino-, Methylbenzylamino-, Methoxybenzylamino-, Dimethoxybenzylamino-, Methylendioxybenzylamino-, Fluorbenzylamino-, Chlorbenzylamino-, Brombenzylamino-, Dichlorbenzylamino-, Trifluormethylbenzylamino-, Methylthiobenzylamino-, 2-(Dimethoxyphenyl)-äthylamino-, Cyclohexylmethylamino-, Furfurylamino-, Pyridylmethylamino-, Phenylamino-, Methoxyphenylamino-, Äthoxyphenylamino-, Fluorphenylamino-, Chlorphenylamino-, Bromphenylamino-, Dimethoxyphenylamino-, Trifluorphenylamino-, Dimethylamino-, Diäthylamino-, Dipropylamino-, Diisopropylamino-, Dibutylamino-, Dicyclopentylamino-, Dicyclohexylamino-, Dicycloheptylamino-, N-Methyl-äthylamino-, N-Äthyl-butylamino-, N-Methyl-cyclohexylamino-, N-Äthyl-cyclohexylamino-, Bis-(2-dimethoxyäthyl)-amino-, N-Methyl-hydroxyäthylamino-, N-(Methyl-(2-methoxyäthyl)-amino-, N-Methylphenylamino-, N-Methyl-trifluorphenylamino-, N-Methylbenzylamino-, N-Methyl-2-(dimethoxy-phenyl)-äthylamino-, N-Methyl-pyridylmethylamino-, Pyrrolidino-, Piperidino-, Methylpiperidino-, Dimethyl-piperidino-, Äthylpiperidino-, Tetrahydropyridino-, Hexamethylenimino-, Heptamethylenimino- oder Furfurylaminogruppe in Betracht.

Bevorzugte Verbindungen der obigen allgemeinen Formel (I) sind jedoch diejenigen, in der

$R_1$ eine gegebenenfalls ab Kohlenstoffatom 2 mit bis zu 5 Hydroxygruppen substituierte Alkylgruppe mit 2 bis 8 Kohlenstoffatomen, eine in 2-Stellung durch eine Methoxy-, Amino-, Diäthylamino- oder Morpholinogruppe substituierte Äthylgruppe, eine Methyl- oder eine Pyridylmethylgruppe,

$R_2$ ein Wasserstoffatom oder ein gegebenenfalls in 2- oder 3-Stellung durch eine Hydroxy- oder Methoxygruppe substituierte Alkylgruppe mit 1 bis 3 Kohlenstoffatomen,

$R_1$ und $R_2$ zusammen mit dem dazwischen liegenden Stickstoffatom eine gegebenenfalls durch eine Hydroxy-, Methoxy-, Hydroxymethyl-, Dimethylamino-, Acetamino-, Aminocarbonyl- oder Äthoxycarbonylgruppe substituierte Piperidinogruppe, eine Morpholino-, 1-Oxido-thiomorpholino- oder Imidazolylgruppe,

$R_3$ eine Morpholino-, Dimethylmorpholino-, Thiomorpholino-, 1-Oxidothiomorpholino- oder 1,1-Dioxidothiomorpholinogruppe und

R eine gegebenenfalls durch eine Phenylgruppe substituierte Alkoxygruppe mit 1 bis 4 Kohlenstoffatomen, eine Alkoxygruppe mit 5 bis 8 Kohlenstoffatomen oder eine Cyclohexylgruppe, eine gegebenenfalls durch eine Phenylgruppe substituierte Alkylthiogruppe mit 1 bis 4 Kohlenstoffatomen oder eine Phenylthiogruppe, wobei die Phenylkerne der vorstehend erwähnten Benzyloxy-, Benzylthio- oder Phenylthiogruppe durch eine Methylendioxy- oder Nitrogruppe oder durch eine Methyl-, Hydroxy-, Methoxy-, Methylthio- oder Trifluormethylgruppe, ein Fluor-, Chlor- oder Bromatom mono- oder disubstituiert sein können, eine Alkylthiogruppe mit 4 bis 8 Kohlenstoffatomen, eine Cyclohexylthio-, 2-Hydroxyäthylthio-, 2-Diäthylaminoäthylthio-, Äthoxy-carbonylmethylthio-, α-Methyl-methylthiobenzylthio-, Naphthylmethylthio-, Furfurylthio- oder Indanylthiogruppe oder eine Gruppe der Formel

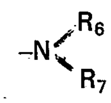 bedeuten, wobei

$R_6$ ein Wasserstoffatom, eine Alkylgruppe mit 1 bis 4 Kohlenstoffatomen oder eine in 2- oder 3-Stellung durch eine Methoxygruppe substituierte Alkylgruppe mit 2 oder 3 Kohlenstoffatomen und

$R_7$ ein Wasserstoffatom, eine gegebenenfalls durch eine Hydroxygruppe substituierte Alkylgruppe mit 2 bis 8 Kohlenstoffatomen, eine in 2- oder 3-Stellung durch eine Methoxy- oder Äthoxygruppe substituierte Alkylgruppe mit 2 oder 3 Kohlenstoffatomen, eine gegebenenfalls durch eine oder 2 Methoxygruppen substituierte Phenylalkylgruppe mit 2 Kohlenstoffatomen im Alkylteil, eine Methyl-, Cyclohexyl-, Cyclohexylmethyl-, 3-Phenylpropyl-, 4-Phenylbutyl-, Furfuryl-, Pyridylmethyl-, Phenyl- oder Benzylgruppe, wobei der Phenylkern der vorstehend erwähnten Phenyl- oder Benzylgruppe durch eine Methylendioxygruppe monosubstituiert oder durch Methyl-, Hydroxy-, Methoxy- Äthoxy-, Methylthio- oder Trifluormethylgruppen, Fluor- und/oder Chloratome mono- oder disubstituiert sein kann, oder

$R_6$ und $R_7$ zusammen mit dem dazwischen liegenden Stickstoffatom eine Pyrrolidino-, Piperidino-, Methylpiperidino-, Äthylpiperidino-, Dimethylpiperidino-, Hexamethylenimino-, Heptamethylenimino- oder Tetrahydropyridinogruppe darstellen.

Besonders bevorzugte Verbindungen sind jedoch diejenigen, in denen

$R_1$ eine ab Kohlenstoffatom 2 gegebenenfalls durch eine oder zwei Hydroxygruppen oder durch eine Methoxygruppe substituierte Alkylgruppe mit 2 bis 5 Kohlenstoffatomen oder eine Methylgruppe,

$R_2$ ein Wasserstoffatom oder eine 2-Hydroxyäthylgruppe,

$R_3$ eine 1-Oxido-thiomorpholinogruppe und

R eine gegebenenfalls durch eine Phenylgruppe substituierte Alkoxy- oder Alkylthiogruppe mit jeweils 1 bis 3 Kohlenstoffatomen, eine Alkoxy- oder Alkylthiogruppe mit jeweils 4 bis 6 Kohlenstoffatomen, eine Cyclohexylthiogruppe oder eine Gruppe der Formel

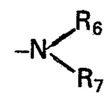 bedeuten, wobei

$R_6$ ein Wasserstoffatom oder eine Alkylgruppe mit 1 bis 4 Kohlenstoffatomen und

$R_7$ eine gegebenenfalls durch eine Hydroxygruppe substituierte Alkylgruppe mit 2 bis 5 Kohlenstoffatomen oder eine durch eine Phenylgruppe substituierte Alkylgruppe mit 1 bis 4 Kohlenstoffatomen, wobei der Phenylkern der oben erwähnten Benzylamino-, Benzyloxy- und Benzylthiogruppe jeweils durch Fluoratome, Chloratome und/oder Methoxygruppen mono- oder disubstituiert sein kann, oder $R_6$ und $R_7$ zusammen mit dem dazwischen liegenden Stickstoffatom eine Pyrrolidino-, Piperidino- oder Hexamethyleniminogruppe darstellen, und deren physiologisch verträgliche Additionssalze mit anorganischen oder organischen Säuren.

Erfindungsgemäss erhält man die neuen Verbindungen nach folgenden Verfahren:

a) Durch Umsetzung eines Pyrimido[5,4-d]pyrimidins der allgemeinen Formel (II)

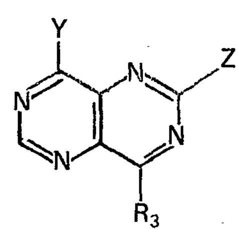

(II)

in der

$R_3$ wie eingangs definiert ist,

Y eine nukleophile Austrittsgruppe darstellt oder die Gruppe R– darstellt, wobei R wie eingangs definiert ist, und

Z eine nukleophile Austrittsgruppe darstellt, mit einem Amin der allgemeinen Formel III

H–A III

in der

A eine Gruppe der Formel

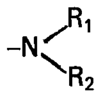 oder 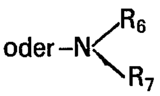 darstellt, wobei $R_1$, $R_2$,

$R_6$ und $R_7$ wie eingangs definiert sind.

Als nukleophile Austrittsgruppe kommt beispielsweise ein Halogenatom wie ein Chlor- oder Bromatom, eine substituierte Hydroxygruppe wie die Phenoxygruppe oder eine Sulfonylgruppe wie die Methylsulfonylgruppe in Bertracht.

Die Umsetzung wird zweckmässigerweise in einem inerten Lösungsmittel wie Aceton, Methyläthylketon, Tetrahydrofuran, Dioxan, Chlorbenzol, Dimethylformamid oder Dimethylsulfoxid gegebenenfalls in Gegenwart einer anorganischen Base, z.B. Natriumkarbonat oder Kaliumhydroxid, oder einer tertiären organischen Base, z.B. Triäthylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 150 °C, vorzugsweise jedoch bei Temperaturen zwischen 20 und 120 °C, durchgeführt. Die Umsetzung kann jedoch auch ohne Lösungsmittel oder in einem Überschuss der eingesetzten Verbindung der allgemeinen Formel (III) durchgeführt werden.

Zur Herstellung von Verbindungen der allgemeinen Formel I, in der R eine Gruppe der Formel $-SR_5$ darstellt:

Umsetzung eines Pyrimido [5,4-d]pyrimidins der allgemeinen Formel (IV)

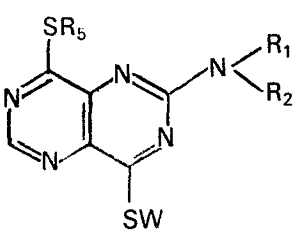

(IV)

in der

$R_1$, $R_2$ und $R_5$ wie eingangs definiert sind und

W eine niedere Alkylgruppe oder eine Aralkylgruppe darstellt, mit einem Amin der allgemeinen Formel (V)

$$H-R_3 \qquad V$$

in der

$R_3$ wie eingangs definiert ist.

Für W kommt beispielsweise die Bedeutung der Methyl-, Äthyl-, Propyl-, Benzyl-, Methylbenzyl-, Chlorbenzyl-, Nitrobenzyl-, oder Naphthylmethylgruppe in Betracht.

Die Umsetzung wird zweckmässigerweise in einem Lösungsmittel wie Benzol, Tetrahydrofuran, Dioxan, Dimethylformamid, Äthanol oder Isopropanol oder in einem Überschuss des eingesetzten Amins der allgemeinen Formel V bei erhöhten Temperaturen, z.B. bei Temperaturen zwischen 60 und 150 °C, vorzugsweise jedoch bei Temperaturen zwischen 70 und 120 °C, durchgeführt. Die Umsetzung kann jedoch auch ohne Lösungsmittel durchgeführt werden.

Erhält man erfindungsgemäss eine Verbindung der allgemeinen Formel (I), in der $R_3$ eine gegebenenfalls durch eine oder zwei Alkylgruppen mit jeweils 1 bis 3 Kohlenstoffatomen substituierte Thiomorpholinogruppe darstellt, so kann diese mittels Oxidation in eine entsprechende Thiomorpholino-1-oxidverbindung übergeführt werden oder eine Verbindung der allgemeinen Formel (I), in der $R_3$ eine gegebenenfalls durch eine oder zwei Alkylgruppen mit jeweils 1 bis 3 Kohlenstoffatomen substituierte Thiomorpholino- oder Thiomorpholino-1-oxidgruppe darstellt, so kann diese mittels Oxidation in eine entsprechende Thiomorpholino-1,1-dioxidverbindung der allgemeinen Formel (I) übergeführt werden.

Die nachträgliche Oxidation wird vorzugsweise in einem Lösungsmittel, z.B. Wasser, Wasser/Pyridin, Eisessig oder Methanol, je nach dem verwendeten Oxidationsmittel zweckmässigerweise bei Temperaturen zwischen −80 und 100 °C durchgeführt.

Zur Herstellung der Thiomorpholino-1-oxide der allgemeinen Formel (I) wird die nachträgliche Oxidation zweckmässigerweise mit einem Äquivalent des verwendeten Oxidationsmittels durchgeführt, mit Wasserstoffperoxid in Eisessig bei 0 bis 20 °C, mit einer Persäure wie Peressigsäure, m-Chlorperbenzoesäure oder Peroxytrifluoressigsäure bei 0 bis 50 °C, mit Kaliumpermanganat in verdünnter Salzsäure bei 0 °C, mit Natriummetaperjodat in wässrigem Methanol oder Äthanol bei 15 bis 25 °C, mit tert.Butylhypochlorit in Methanol bei −80 bis −30 °C, mit Jodbenzoldichlorid in wässrigem Pyridin bei 0 bis 50 °C, mit Salpetersäure in Eisessig bei 0 bis 20 °C und mit Chromsäure in Eisessig oder Aceton bei 0 bis 20 °C.

Zur Herstellung der Thiomorpholino-1,1-dioxide der allgemeinen Formel (I) wird die nachträgliche Oxidation zweckmässigerweise mit zwei Äquivalenten des betreffenden Oxidationsmittels ausgehend von einer Thiomorpholinoverbindung der allgemeinen Formel (I) bzw. mit einem Äquivalent ausgehend von einer Thiomorpholino-1-oxidverbindung der allgemeinen Formel (I) analog wie oben beschrieben durchgeführt. Die Umsetzung wird jedoch bei einer um 10–50 °C höheren Reaktionstemperatur durchgeführt.

Desweiteren lassen sich die neuen Verbindungen der allgemeinen Formel (I) in ihre physiologisch verträglichen Säureadditionssalze mit anorganischen oder organischen Säuren überführen. Als Säuren haben sich beispielsweise Salzsäure, Bromwasserstoffsäure, Schwefelsäure, Phosphorsäure, Methansulfonsäure, p-Toluolsulfonsäure, Essigsäure, Milchsäure, Zitronensäure, Weinsäure, Bernsteinsäure, Maleinsäure, Fumarsäure oder Salicylsäure als geeignet erwiesen.

Die als Ausgangsstoffe verwendeten Verbindungen der allgemeinen Formeln (II) bis (V) sind teilweise literaturbekannt bzw. erhält man nach literaturbekannten Verfahren. So erhält man beispielsweise die als Ausgangstoffe verwendeten Verbindungen der allgemeinen Formel (II) und (IV) durch stufenweisen Ersatz der Chloratome des 2,4,8-Trichlor-pyrimido[5,4-d]pyrimidins (siehe DE-C-1 116 676).

Wie bereits eingangs erwähnt, weisen die erfindungsgemäss hergestellten neuen Verbindungen der allgemeinen Formel (I) und deren physiologisch verträgliche Säureadditionssalze wertvolle pharmakologische Eigenschaften auf, insbesondere antithrombotische Eigenschaften. Ausserdem weisen sie neben einer blutdrucksenkenden und cardiotonischen Wirkung eine PDE-Hemmwirkung und eine Hemmwirkung auf die Aggregation von in die Blutbahn geschwemmten Krebszellen auf.

Beispielsweise wurden die folgenden Verbindungen auf ihre biologischen Eigenschaften untersucht:

A = 2-(2-Hydroxyäthylamin) -4- (1-oxido-thiomorpholino) -8- propyl-thio-pyrimido[5,4-d]pyrimidin,

B = 8-Cyclohexylthio -2- (2-hydroxyäthylamino) -4(1-oxido-thio-morpholino) -pyrimido[5,4-d]pyrimidin,

C = 8-Benzylthio -2- (2-hydroxyäthylamino) -4- (1-oxido-thiomorpholino) -pyrimido[5,4-d] pyrimidin,

D = 8-Benzylthio -2- diäthanolamino -4- (1-oxido-thiomorpholino)-pyrimido[5,4-d]pyrimidin,

E = 8-Benzylthio -2[N-(2-hydroxyäthyl) -2-methoxyäthylamino]-4-(1-oxido-thiomorpholino) -pyrimido[5,4-d]pyrimidin,

F = 2-(2-Hydroxyäthylamino) -8- (α-methyl -4- methylthio-benzylthio)-4-(1-oxido-thiomorpholino)-pyrimido[5,4-d]pyrimidin,

G = 8-Furfurylthio -2- (2-hydroxyäthylamino) -4- (1-oxido-thiomorpholino)-pyrimido[5,4-d] pyrmidin,

H = 8-(N-Äthyl-butylamino) -2(2-hydroxyäthylamino) -4- (1-oxidothiomorpholino)-pyrimido[5,4-d]pyrimidin,

I = 2-(2-Hydroxyäthylamino) -4- (1-oxido-thiomorpholino) -8- (L-1-phenyl-äthylamino) -pyrimido[5,4-d]pyrimidin,

K = 8-(4-Chlorbenzylamino) -2- (2-hydroxyäthylamino) -4- (1-oxido-thiomorpholino) -pyrimido[5,4-d]pyrimidin,

L = 2-Diäthanolamino -4- (1-oxido-thiomorpholino) -8- piperidino-pyrimido[5,4-d]pyrimidin,

M = 2-Diäthanolamino -8- isopentoxy -4- (1-oxido-thiomorpholino)-pyrimido[5,4-d]pyrimidin,

N = 8-Benzyloxy -2- diäthanolamino -4- (1-oxido-thiomorpholino)-pyrimido[5,4-d]pyrimidin,

O = 8-Benzyloxy -2- [N-(2-hydroxyäthyl) -2-methoxyäthylamino] -4(1-oxido-thiomorpholino)-pyrimido[5,4-d]pyrimidin und

P = 2-Diäthanolamino -4- (1-oxido-thiomorpholino) -8- (3-phenyl-propoxy)-pyrimido[5,4-d]pyrimidin

1. Antithrombotische Wirkung:

Methodik:

Die Thrombozytenaggregation wird nach der Methode von BORN und CROSS (J. Physiol. 170, 397 (1964) ) in plättchenreichem Plasma gesunder Versuchspersonen gemessen. Zur Gerinnungshemmung wird das Blut mit Natriumcitrat 3,14% im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die grösste Lichtdurchlässigkeit vorliegt, dient zur Berechnung der «optical density».

Die Collagen-Menge wird möglichst gering gewählt, aber doch so, dass sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Messzahlen wird graphisch eine $EC_{50}$ berechnet, die sich auf eine 50%ige Änderung der «optical density» im Sinne einer Aggregationshemmung bezieht.

Die nachfolgende Tabelle enthält die gefundenen Ergebnisse:

| Substanz | $EC_{50}$ µMol/1 |
|---|---|
| A | 0,31 |
| B | 0,048 |
| C | 0,021 |
| D | 0,062 |
| E | 0,052 |
| F | 0,029 |
| G | 0,052 |
| H | 0,3 |
| I | 0,028 |
| K | 0,28 |
| L | 0,69 |
| M | 0,46 |
| N | 0,23 |
| O | 0,3 |
| P | 0,36 |

2. Akute Toxizität:

Die akute Toxizität der zu untersuchenden Substanzen wurde orientierend an Gruppen von 6 bis 10 Mäusen nach oraler Gabe einer Dosis von 250 mg/kg bestimmt (Beobachtungszeit: 14 Tage). Hierzu wurden die Substanzen in 2%iger Methylcellulose suspendiert, anschliessend wurde etwas Wasser zugegeben und die jeweilige Substanz den wachen Tieren per Schlundsonde appliziert.

Die folgende Tabelle enthält die gefundenen Werte:

| Substanz | Toxizität in mg/kg p.o. |
|---|---|
| A | > 250 (0 von 6 Tieren gestorben) |
| B | > 250 (0 von 10 Tieren gestorben) |
| C | > 250 (0 von 10 Tieren gestorben) |
| D | > 250 (o von 10 Tieren gestorben) |
| E | > 250 (0 von 10 Tieren gestorben) |
| F | > 250 (0 von 6 Tieren gestorben) |
| G | > 250 (0 von 6 Tieren gestorben) |
| H | > 250 (0 von 6 Tieren gestorben) |
| K | > 250 (0 von 10 Tieren gestorben) |
| L | > 250 (0 von 10 Tieren gestorben) |
| M | > 250 (0 von 6 Tieren gestorben) |
| N | > 250 (0 von 10 Tieren gestorben) |
| O | > 250 (0 von 6 Tieren gestorben) |
| P | > 250 (0 von 6 Tieren gestorben) |

Auf Grund ihrer pharmakologischen Eigenschaften eignen sich die Verbindungen der allgemeinen Formel (I) sowie deren physiologisch verträgliche Säureadditionssalze mit anorganischen oder organischen Säuren zur Prophylaxe thrombo-embolischer Erkrankungen wie Coronarinfarkt, Cerebralinfarkt, sogn. transient ischaemic attacks, Amaurosis fugax und zur Prophylaxe der Arteriosklerose und der Metastasenbildung. Hierzu lassen sich diese, gegebenenfalls in Kombination mit anderen Wirksubstanzen, in die üblichen pharmazeutischen Zubereitungsformen wie Dragées, Tabletten, Kapseln, Suppositorien, Lösungen oder Suspensionen einarbeiten. Die Einzeldosis am Erwachsenen beträgt hierbei 0,1 – 20 mg, vorzugsweise 0,5 – 5 mg, 2 – 4 × täglich; somit beträgt die Tagesdosis 0,2 – 80 mg.

Die nachfolgenden Beispiele sollen die Erfindung näher erläutern:

Vorbemerkung:

Bei den Schmelzpunktangaben handelt es sich um unkorrigierte Schmelzpunkte.

Beispiele zur Herstellung der Ausgangsverbindungen:

Beispiel A

2,8-Dichlor-4-morpholino-pyrimido[5,4-d]pyrimidin

118 g (0,5 Mol) 2,4,8-Trichlor-pyrimido[5,4-d]pyrimidin werden in 1,2 l Aceton suspendiert und anschliessend unter Rühren bei Raumtemperatur eine Lösung von 44 ml (0,5 Mol) Morpholin und 70 ml (0,5 Mol)Triäthylamin in 100 ml Aceton langsam zulaufen gelassen. Nach anschliessendem etwa halbstündigem Rühren gibt man zum Reaktionsgemisch 1,3 l Wasser, wobei sich das Triäthylaminhydrochlorid auflöst und weiteres Reaktionsprodukt abscheidet. Nach einigem Stehen wird abgesaugt, der Niederschlag gut mit Wasser und dann mit etwas Methanol gewaschen und bei 60 °C getrocknet.

Ausbeute: 132 g (92% der Theorie) vom Schmelzpunkt 179–181 °C,

Schmelzpunkt: 183–185 °C (aus Äthanol)

Die Umsetzung kann in völlig analoger Weise auch unter Verwendung einer wässrigen Kaliumcarbonat-Lösung anstelle von Triäthylamin durchgeführt werden.

Analog Beispiel A wurden folgende Verbindungen hergestellt:

2,8-Dichlor-4-(2-methylmorpholino)-pyrimido[5,4-d]pyrimidin Schmelzpunkt: 129–131 °C

2,8-Dichlor-4-(2,6-dimethylmorpholino)-pyrimido[5,4-d]pyrimidin Schmelzpunkt: 181–183 °C

2,8-Dichlor-4-thiomorpholino-pyrimido[5,4-d]pyrimidin Schmelzpunkt: 154–157 °C

2,8-Dichlor-4-(1-oxido-thiomorpholino)-pyrimido[5,4-d]pyrimidin Schmelzpunkt: 195–198 °C (Dioxan)

2,8-Dichlor-4-(1,1-dioxido-thiomorpholino)-pyrimido[5,4-d]pyrimidin

Schmelzpunkt: 270–273 °C (Zers., Dioxan).

Beispiel B

8-Benzylthio-2-chlor-4-morpholino-pyrimido[5,4-d]pyrimidin

Unter Rühren wird zu einer Suspension von 143 g (0,5 Mol) 2,8-Dichlor-4-morpholino-pyrimido[5,4-d]pyrimidin in 2 l Aceton eine Lösung von 23 g (0,52 Mol) Natriummethylat in 150 ml Methanol und 59 ml (0,5 Mol) Benzylmercaptan bei Raumtemperatur langsam zulaufen gelassen. Anschliessend wird noch etwa eine Stunde lang gerührt und dann zum Reaktionsgemisch etwa 2 l Wasser gegeben, wobei sich das ausgefallene Natriumchlorid löst und weiteres Reaktionsprodukt abscheidet. Nach einigem Stehen wird abgesaugt, mit etwa 1 l Wasser und anschliessend mit ca. 500 ml Methanol gewaschen und bei 60 °C getrocknet.

Ausbeute: 182 g (97% der Theorie) vom Schmelzpunkt 157–159 °C, Schmelzpunkt: 159–161 °C (aus Isopropanol).

Die Reaktion kann in völlig analoger Weise auch unter Verwendung von 2n-Natronlauge anstelle von Natriummethylat-Lösung ausgeführt werden.

Beispiel C

8-(N-Benzyl-methylamino)-2-chlor-4(1-oxido-thiomorpholino)-pyrimido[5,4-d]pyrimidin

Zu einer Suspension von 15,9 g (0,05 Mol) 2,8-Dichlor-4-(1-oxido-thiomorpholino)-pyrimido[5,4-d]pyrimidin in ca. 250 ml Dioxan wird unter Rühren eine Lösung von 12,2 g (0,1 Mol) N-Benzyl-methylamin in 50 ml Dioxan langsam eingegossen und anschliessend das Ganze noch etwa 30 Minuten lang auf 30–40 °C erwärmt. Beim Aufnehmen des Reaktionsgemisches in etwa 1 l Wasser scheidet sich das Reaktionsprodukt als schwach gelblicher Niederschlag ab. Nach einigem Stehen wird abgesaugt, mit Wasser gewaschen und bei etwa 60 °C getrocknet.

Ausbeute: 18,6 g (92% der Theorie).

Nach Umkristallisieren aus Äthanol schmilzt das 8-(N-Benzyl-methylamino)-2-chlor-4-(1-oxido-thiomorpholino)-pyrimido-[5,4-d]pyrimidin bei 158–160 °C.

Beispiel D

8-Benzyloxy-2-chlor-4-(1-oxido-thiomorpholino)-pyrimido[5,4-d]pyrimidin

Zu einer etwa 30 °C warmen Lösung von 15,9 g (0,05 Mol) 2,8-Dichlor-4-(1-oxido-thiomorpholino)-pyrimido[5,4-d]pyrimidin in 400 ml Dioxan wird eine aus 22 g (0,2 Mol) Benzylalkohol in 100 ml absolutem Benzol mit 1,6 g (0,07 Mol) Natrium hergestellte Alkoholatlösung gegeben und das erhaltene Gemisch etwa zwei Stunden lang bei Raumtemperatur gerührt. Anschliessend wird das Reaktionsgemisch im Vakuum eingeengt und der Rückstand mit etwa 500 ml Wasser versetzt. Der nach kurzem Stehen fest gewordene Niederschlag wird abgesaugt, mit Wasser gewaschen und bei etwa 70 °C getrocknet.

Ausbeute: 16 g (82% der Theorie).

Nach Umkristallisieren aus Dioxan schmilzt das 8-Benzyloxy-2-chlor-4-(1-oxido-thiomorpholino)-pyrimido[5,4-d]pyrimidin bei 227–229 °C.

Analog den Beispielen B, C und D werden alle in den folgenden Beispielen für die Herstellung der Endprodukte angegebenen Ausgangsverbindungen hergestellt.

Herstellung der Endprodukte:

Beispiel 1

8-Benzylthio -2- (2-hydroxyäthylamino)- 4- (1-oxido-thiomorpholino)-pyrimido[5,4-d]pyrimidin

20,3 g (0,05 Mol) 8-Benzylthio-2-chlor-4-(1-oxido-thiomorpholino)-pyrimido[5,4-d]pyrimidin (Schmelzpunkt: 188–190 °C) werden mit 8 g (0,13 Mol) 2-Hydroxyäthylamin in 400 ml Dioxan etwa 45 Minuten lang unter Rückfluss erhitzt. Das Lösungsmittel wird im Vakuum grösstenteils abdestilliert und der verbleibende Rückstand in etwa 500 ml Wasser aufgenommen. Das nach einigem Stehen abgeschiedene Reaktionsprodukt wird abgesaugt, mit Wasser gewaschen und bei etwa 70 °C getrocknet.

Ausbeute: 20,8 g (97% der Theorie).

Nach Umkristallisieren aus Äthanol/Dioxan schmilzt das 8-Benzylthio-2-(2-hydroxyäthylamino)-4-(1-oxido-thiomorpholino)-pyrimido-[5,4-d]pyrimidin bei 215–217 °C.

$C_{19}H_{22}N_6O_2S_2$ (430,6)

| | | | | |
|---|---|---|---|---|
| Ber.: | C 52,99 | H 5,16 | N 19,52 | S 14,89 |
| Gef.: | 53,00 | 5,23 | 19,56 | 14,70 |

Die gleiche Verbindung erhält man auch durch Umsetzung von 4,8-Bis(benzylthio)-2-(2-hydroxyäthylamino) -pyrimido [5,4-d] pyrimidin (Schmelzpunkt: 154–155 °C) mit Thiomorpholin-1-oxis bei 80 bis 100 °C in Gegenwart von Thiomorpholin-1-oxid-hydrochlorid oder durch Oxidation von 8-Benzylthio-2-(2-hydroxyäthylamino)-4-thiomorpholino-pyrimido[5,4-d]pyrimidin (Schmelzpunkt: 175–177 °C)

a) mit Natriummetaperjodat in Methanol unter Rückfluss,

b) mit Wasserstoffperoxid in Eisessig bei etwa 30 °C und

c) mit Kaliumpermanganat in 1 n-Salzsäure bei etwa 30 °C.

Beispiel 2

8- (N-Benzyl-methylamino) -2- (2-hydroxyäthylamino) -4- (1-oxido-thiomorpholino)-pyrimido[5,4-d]pyrimidin

8,1 g (0,02 Mol) 8-(N-Benzyl-methylamino)-2-chlor-4-(1-oxido-thiomorpholino)-pyrimido[5,4-d]pyrimidin (Schmelzpunkt: 158–160 °C) werden mit 12,2 g (0,2 Mol) 2-Hydroxyäthylamin eine Stunde lang auf etwa 90 °C erhitzt. Das erhaltene Gemisch wird in etwa 250 ml Wasser aufgenommen, wobei sich das Reaktionsprodukt als bald erstarrender Niederschlag abscheidet. Es wird abgesaugt, mit Wasser gewaschen und bei etwa 60 °C getrocknet. Ausbeute: 8,2 g (96% der Theorie).

Nach einmaligem Umkristallisieren aus Äthanol schmilzt das 8-(N-Benzyl-methylamino)-2-(2-hydroxyäthylamino)-4-(1-oxido-thiomorpholino)-pyrimido[5,4-d]pyrimidin bei 181–182 °C.

$C_{20}H_{25}N_7O_2S$ (427,5)

| | | | | |
|---|---|---|---|---|
| Ber.: | C 56,19 | H 5,89 | N 22,48 | S 7,50 |
| Gef.: | 56,30 | 5,78 | 22,45 | 7,32 |

Beispiel 3

2-Diäthanolamino-4-(1-oxido-thiomorpholino)-8-piperidino-pyrimido[5,4-d]pyrimidin

11,0 g (0,03 Mol) 2-Chlor-4-(1-oxido-thiomorpholino)-8-piperidino-pyrimido[5,4-d]pyrimidin (Schmelzpunkt: 203–204 °C) werden mit 32 g (0,3 Mol) Diäthanolamin etwa 30 Minuten lang auf 120 °C erhitzt und das erhaltene Gemisch anschliessend in etwa 300 ml Wasser aufgenommen. Das nach kurzer Zeit kristallin abgeschiedene Reaktionsprodukt wird abgesaugt, mit Wasser gewaschen und getrocknet.

Ausbeute: 12,4 g (95% der Theorie).

Nach Umkristallisieren aus Äthanol schmilzt das 2-Diäthanolamino-4-(1-oxido-thiomorpholino)-8-piperidino-pyrimido[5,4-d]pyrimidin bei 115–118 °C.

$C_{19}H_{29}N_7O_3S$ (435,6)

| | | | | |
|---|---|---|---|---|
| Ber.: | C 52,39 | H 6,71 | N 22,51 | S 7,36 |
| Gef.: | 53,03 | 6,70 | 22,63 | 7,09 |

Beispiel 4

8-Benzyloxy-2-diäthanolamino-4-(1-oxido-thiomorpholino)-pyrimido[5,4-d]pyrimidin

7,8 g (0,02 Mol) 8-Benzyloxy-2-chlor-4-(1-oxido-thiomorpholino)-pyrimido[5,4-d]pyrimidin (Schmelzpunkt: 227–229 °C) werden mit 8,4 g (0,08 Mol) Diäthanolamin in 100 ml Dioxan oder auch ohne Lösungsmittel etwa 1 Stunde lang auf 100 °C erhitzt. Das Lösungsmittel wird im Vakuum abdestilliert und der verbleibende Rückstand in etwa 300 ml Wasser aufgenommen. Das nach einigem Stehen erstarrte Reaktionsprodukt wird abgesaugt, mit Wasser gewaschen und bei etwa 60 °C getrocknet.

Ausbeute: 8,4 g (92% der Theorie).

Nach Umkristallisieren aus Äthanol schmilzt das 8-Benzyloxy-2-diäthanolamino-4-(1-oxido-thiomorpholino)-pyrimido[5,4-d]pyrimidin bei 175–177 °C.

$C_{21}H_{26}N_6O_4S$ (458,6)

| | | | | |
|---|---|---|---|---|
| Ber.: | C 55,01 | H 5,72 | N 18,33 | S 6,99 |
| Gef.: | 55,30 | 5,80 | 18,55 | 6,48 |

Beispiel 5

2- (2-Hydroxyäthylamino) -8- methylthio -4- (1-oxido-thiomorpholino)-pyrimido[5,4-d]pyrimidin

Hergestellt analog Beispiel 1 aus 2-Chlor-8-methylthio-4-(1-oxido-thiomorpholino)-pyrimido[5,4-d]pyrimidin (Schmelzpunkt: 260–262 °C) und 2-Hydroxyäthylamin.

Schmelzpunkt: 168–170 °C (Methanol).

Beispiel 6

2-Diäthanolamino -8- methylthio -4- (1-oxido-thiomorpholino) -pyrimido[5,4-d]pyrimidin

Hergestellt analog Beispiel 3 aus 2-Chlor-8-methylthio-4-(1-oxido-thiomorpholino)-pyrimido[5,4-d]pyrimidin und Diäthanolamin.

Schmelzpunkt: 228–230 °C (Dimethylformamid).

Beispiel 7

2- (2-Hydroxyäthylamino) -4- (1-oxido-thiomorpholino) -8- propylthio-pyrimido[5,4-d]pyrimidin

Hergestellt analog Beispiel 1 aus 2-Chlor -4- (1-oxido-thiomorpholino) -8- propylthio-pyrimido[5,4-d]pyrimidin (Schmelzpunkt: 209–210°C) und 2-Hydroxyäthylamin.

Schmelzpunkt: 170–172 °C (Äthanol).

Beispiel 8

2-(2-Hydroxyäthylamino)-8-(3-methyl-n- butyl)-thio-4-(1-oxido-thiomorpholino)-pyrimido-[5,4-d]pyrimidin

Hergestellt analog Beispiel 1 aus 2-Chlor-8-(3-methyl-n-butyl)-thio-4-(1-oxido-thiomorpholino)-pyrimido[5,4-d]pyrimidin (Schmelzpunkt: 175–177 °C) und 2-Hydroxyäthylamin.

Schmelzpunkt: 200–202 °C (Äthanol).

Beispiel 9

2-(2-Hydroxyäthylamino)-8-octylthio-4-(1-oxido-thiomorpholino)-pyrimido[5,4-d]pyrimidin

Hergestellt analog Beispiel 1 aus 2-Chlor-8-octylthio -4- (1-oxido-thiomorpholino) -pyrimido[5,4-d]pyrimidin (Schmelzpunkt: 106–108 °C) und 2-Hydroxyäthylamin.

Schmelzpunkt: 158–160 °C (Äthanol).

Beispiel 10

8-Cyclohexylthio -2- (2-hydroxyäthylamino) -4- (1-oxido-thiomorpholino) -pyrimido[5,4-d]pyrimidin

Hergestellt analog Beispiel 1 aus 2-Chlor-8-cyclohexylthio-4-(1-oxido-thiomorpholino)-pyrimido[5,4-d]pyrimidin (Schmelzpunkt: 225–227 °C) und 2-Hydroxyäthylamin.

Schmelzpunkt: 204–206 °C (Äthanol).

Beispiel 11

8-Benzylthio-2-(2-hydroxyäthylamino)-4-morpholino-pyrimido-[5,4-d]pyrimidin

Hergestellt analog Beispiel 1 aus 8-Benzylthio-2-chlor-4-morpholono-pyrimido[5,4-d]pyrimidin (Schmelzpunkt: 159–161 °C) und 2-Hydroxyäthylamin durch Erwärmen in Dimethylsulfoxid auf 50 °C. Schmelzpunkt: 151–153 °C (Essigsäureäthylester).

Die gleiche Substanz erhält man in analoger Weise auch durch Erhitzen von 8-Benzylthio-4-morpholino-2-phenoxy-pyrimido[5,4-d]pyrimidin (Schmelzpunkt: 159–161 °C) mit 2-Hydroxyäthylamin auf etwa 90 °C.

Beispiel 12

8-Benzylthio-2-(2-hydroxyäthylamino)-4-thiomorpholino-pyrimido-[5,4-d]pyrimidin

Hergestellt analog Beispiel 1 aus 8-Benzylthio-2-chlor-4-thiomorpholino-pyrimido[5,4-d]pyrimidin (Schmelzpunkt: 175–177 °C) und 2-Hydroxyäthylamin.

Schmelzpunkt: 155–157 °C (Äthanol/Dioxan).

Beispiel 13

8-Benzylthio-4-(1,1-dioxido-thiomorpholino)-2-(2-hydroxyäthylamino)-pyrimido[5,4-d]pyrimidin

Hergestellt analog Beispiel 1 aus 8-Benzylthio-2-chlor-4-(1,1-dioxido-thiomorpholino)-pyrimido[5,4-d]pyrimidin (Schmelzpunkt: 230–233°C) und 2-Hydroxyäthylamin.

Schmelzpunkt: 193–195 °C (Essigsäureäthylester).

Beispiel 14

8-Benzylthio-2-diäthanolamino-4-(1-oxido-thiomorpholino)-pyrimido[5,4-d]pyrimidin

Hergestellt analog Beispiel 1 aus 8-Benzylthio-2-chlor-4-(1-oxido-thiomorpholino)-pyrimido[5,4-d]pyrimidin (Schmelzpunkt: 188–190 °C) und Diäthanolamin durch dreistündiges Kochen unter Rückfluss.

Schmelzpunkt: 186–188 (Äthanol).

Beispiel 15

8-Benzylthio-2-[N- (2-hydroxyäthyl)-methylamino]4- (1-oxidothiomorpholino)- pyrimido-[5,4-d]pyrimidin

Hergestellt analog Beispiel 1 aus 8-Benzylthio-2-chlor -4- (1-oxido-thiomorpholino)-pyrimido[5,4-d]pyrimidin und N-Methyläthanolamin.

Schmelzpunkt: 177–179 °C (Äthanol).

Beispiel 16

8-Benzylthio -2- [N-(2-hydroxyäthyl)- 2-methoxyäthylamino]- 4-(1-oxido-thiomorpholino)-pyrimido[5,4-d]pyrimidin

Hergestellt analog Beispiel 1 aus 8-Benzylthio-2-chlor -4- (1-oxido-thiomorpholino)-pyrimido[5,4-d]pyrimidin und N-Methoxyäthyl-äthanolamin.

Schmelzpunkt: 136–138 °C (Essigsäureäthylester).

Beispiel 17

8-Benzylthio -2- (3-hydroxypropylamino)- 4-(1-oxido-thiomorpholino)- pyrimido[5,4-d]pyrimidin

Hergestellt analog Beispiel 1 aus 8-Benzylthio-2-chlor-4(1-oxido-thiomorpholino)- pyrimido-

[5,4-d]pyrimidin und 3-Amino-1-propanol.
Schmelzpunkt: 148–150 °C (Äthanol).

Beispiel 18
8-Benzylthio-2-(2-hydroxypropylamino)-4-(1-oxido-thiomorpholino)-pyrimido[5,4-d]pyrimidin
Hergestellt analog Beispiel 1 aus 8-Benzylthio-2-chlor-4-(1-oxido-thiomorpholino)-pyrimido[5,4-d]pyrimidin und 1-Amino-2-propanol.
Schmelzpunkt: 175–177 °C (Äthanol).

Beispiel 19
8-Benzylthio-2-(2,3-dihydroxypropylamino)-4-(1-oxido-thiomorpholino)pyrimido[5,4-d]-pyrimidin
Hergestellt analog Beispiel 1 aus 8-Benzylthio-2-chlor-4-(1-oxido-thiomorpholino)-pyrimido-[5,4-d]pyrimidin und 1-Amino-2,3-propandiol.
Schmelzpunkt: 167–169 °C (Äthanol).

Beispiel 20
8-Benzylthio-2-(5-hydroxypentylamino)-4-(1-oxido-thiomorpholino)-pyrimido[5,4-d]pyrimidin
Hergestellt analog Beispiel 1 aus 8-Benzylthio-2-chlor-4-(1-oxido-thiomorpholino)pyrimido-[5,4-d]pyrimidin und 5-Amino-1-pentanol.
Schmelzpunkt: 158–159 °C (Äthanol).

Beispiel 21
8-Benzylthio-2-(6-hydroxyhexylamino)-4-(1-oxido-thiomorpholino)-pyrimido[5,4-d]pyrimidin
Hergestellt analog Beispiel 1 aus 8-Benzylthio-2-chlor-4(1-oxido-thiomorpholino)-pyrimido-[5,4-d]pyrimidin und 6-Amino-1-hexanol.
Schmelzpunkt: 125–127 °C (Äthanol).

Beispiel 22
8-Benzylthio-2-(N-methyl-D-glucamino)-4-(1-oxido-thiomorpholino)-pyrimido[5,4-d]pyrimidin
Hergestellt analog Beispiel 1 aus 8-Benzylthio-2-chlor-4-(1-oxido-thiomorpholino)-pyrimido-[5,4-d] pyrimidin und N-Methyl-D-glucamin.
Schmelzpunkt: 203–205 °C (Dioxan).

Beispiel 23
8-Benzylthio-2-methylamino-4-(1-oxido-thiomorpholino)-pyrimido[5,4-d]pyrimidin
Hergestellt analog Beispiel 1 aus 8-Benzylthio-2-chlor-4-(1-oxido-thiomorpholino)-pyrimido-[5,4-d] pyrimidin und Methylamin in Dioxan bei etwa 100 °C unter Druck.
Schmelzpunkt: 190–192 °C (Äthanol/Dioxan).

Beispiel 24
8-Benzylthio-4-(1-oxido-thiomorpholino)2-propylamino-pyrimido-[5,4-d]pyrimidin
Hergestellt analog Beispiel 23 aus 8-Benzylthio-2-chlor-4-(1-oxido-thiomorpholino)-pyrimido[5,4-d] pyrimidin und Propylamin.
Schmelzpunkt: 181–182 °C (Äthanol/Dioxan).

Beispiel 25
8-Benzylthio-2-(3-methyl-n-butyl)amino-4-(1-oxido-thiomorpholino)-pyrimido[5,4-d]pyrimidin
Hergestellt analog Beispiel 1 aus 8-Benzylthio-2-chlor-4-(1-oxido-thiomorpholino)-pyrimido[5,4-d]pyrimidin und 3-Methyl-n-butylamin.
Schmelzpunkt: 195–197 °C (Äthanol/Dioxan).

Beispiel 26
2-(2-Aminoäthylamino)-8-benzylthio-4-(1-oxido-thiomorpholino)-pyrimido[5,4-d]pyrimidin
Hergestellt analog Beispiel 1 aus 8-Benzylthio-2-chlor-4-(1-oxido-thiomorpholino)-pyrimido[5,4-d]pyrimidin und Äthylendiamin.
Schmelzpunkt: 174–176 °C (Äthanol).

Beispiel 27
8-Benzylthio-2-(2-diäthylamino-äthylamino)-4-(1-oxido-thiomorpholino)-pyrimido[5,4-d]-pyrimidin
Hergestellt analog Beispiel 1 aus 8-Benzylthio-2-chlor-4-(1-oxido-thiomorpholino)-pyrimido[5,4-d]pyrimidin und 2-Diäthylamino-äthylamin.
Schmelzpunkt: 112–115 °C (Äthanol).

Beispiel 28
8-Benzylthio-2-(2-morpholino-äthylamino)-4-(1-oxido-thiomorpholino)-pyrimido[5,4-d) pyrimidin
Hergestellt analog Beispiel 1 aus 8-Benzylthio-2-chlor-4-(1-oxido-thiomorpholino)pyrimido-[5,4-d]pyrimidin und 2-Morpholinoäthylamin.
Schmelzpunkt: 187–190 °C.

Beispiel 29
8-Benzylthio-4-(1-oxido-thiomorpholino)-2-(3-picolylamino)-pyrimido[5,4-d]pyrimidin
Hergestellt analog Beispiel 1 aus 8-Benzylthio-2-chlor-4-(1-oxido-thiomorpholino)pyrimido-[5,4-d]pyrimidin und 3-Picolylamin.
Schmelzpunkt: 145–148 °C (Äthanol).

Beispiel 30
8-Benzylthio-2-(N-methyl-3-picolylamino)-4-(1-oxido-thiomorpholino)-pyrimido-[5,4-d]pyrimidin
Hergestellt analog Beispiel 1 aus 8-Benzylthio-2-chlor-4-(1-oxido-thiomorpholino)-pyrimido[5,4-d]pyrimidin und N-Methyl-3-picolylamin.
Schmelzpunkt: 186–188 °C (Äthanol).

Beispiel 31
8-Benzylthio-2-(3-hyroxypiperidino)-4-(1-oxido-thiomorpholino)-pyrimido-[5,4-d]pyrimidin
Hergestellt analog Beispiel 1 aus 8-Benzylthio-2-chlor-4-(1-oxido-thiomorpholino)-pyrimido-[5,4-d]pyrimidin und 3-Hydroxypiperidin.
Schmelzpunkt: 239–241 °C (Äthanol).

Beispiel 32
8-Benzylthio -2- (4-hydroxypiperidino) -4- (1-oxido-thiomorpholino)pyrimido[5,4-d]pyrimidin
Hergestellt analog Beispiel 1 aus 8-Benzylthio-2-chlor -4- (1-oxido-thiomorpholino)-pyrimido-[5,4-d] pyrimidin und 4-Hydroxypiperidin.
Schmelzpunkt: 247–249 °C (Äthanol).

Beispiel 33
8-Benzylthio -2- (4-methoxypiperidino) -4- (1-oxido-thiomorpholino)- pyrimido-[5,4-d]pyrimidin
Hergestellt analog Beispiel 1 aus 8-Benzylthio-2-chlor-4- (1-oxido-thiomorpholino)- pyrimido-[5,4-d]pyrimidin und 4-Methoxypiperidin.
Schmelzpunkt: 196–199 °C (Äthanol).

Beispiel 34
8-Benzylthio -2- (2-hydroxymethylpiperidino) -4- (1-oxido-thiomorpholino) -pyrimido-[5,4-d]pyrimidin.
Hergestellt analog Beispiel 1 aus 8-Benzylthio-2-chlor-4- (1-oxido-thiomorpholino)- pyrimido-[5,4-d]pyrimidin und 2-Hydroxymethyl-piperidin.
Schmelzpunkt: 190–192 °C (Äthanol).

Beispiel 35
8-Benzylthio -2- (3-hydroxymethylpiperidino) -4-(1-oxido-thiomorpholino)- pyrimido[5,4-d]-pyrimidin
Hergestellt analog Beispiel 1 aus 8-Benzylthio-2-chlor -4- (1-oxido-thiomorpholino)- pyrimidin[5,4-d]pyrimidin und 3-Hydroxymethyl-piperidin.
Schmelzpunkt: 222–223 °C (Dioxan).

Beispiel 36
8-Benzylthio -2- (4-dimethylamino-piperidino) -4-(1-oxido-thiomorpholino)- pyrimido[5,4-d]-pyrimidin
Hergestellt analog Beispiel 1 aus 8-Benzylthio-2-chlor -4- (1-oxido-thiomorpholino)pyrimido-[5,4-d]pyrimidin und 4-Dimethylamino-piperidin.
Schmelzpunkt: 202–204 °C (Äthanol/Dioxan).

Beispiel 37
2-(4-Acetylamino-piperidino) -8-benzylthio -4- (1-oxido-thiomorpholino)- pyrimido-[5,4-d]pyrimidin
Hergestellt analog Beispiel 1 aus 8-Benzylthio-2-chlor -4- (1-oxido-thiomorpholino)- pyrimido[5,4-d]pyrimidin und 4-Acetylamino-piperidin.
Schmelzpunkt: 295–297 °C.

Beispiel 38
8-Benzylthio -2- (4-carbamoyl-piperidino) -4- (1-oxido-thiomorpholino)- pyrimido-[5,4-d]pyrimidin
Hergestellt analog Beispiel 1 aus 8-Benzylthio-2-chlor-4- (1-oxido-thiomorpholino)pyrimido-[5,4-d]pyrimidin und Piperidin-4-carbonsäureamid.
Schmelzpunkt: 270–273 °C.

Beispiel 39
2-(4-Äthoxycarbonyl-piperidino) -8-benzylthio -4-(1-oxido-thiomorpholino)- pyrimido[5,4-d]-pyrimidin
Hergestellt analog Beispiel 1 aus 8-Benzylthio-2-chlor -4- (1-oxido-thiomorpholino)-pyrimido[5,4-d]pyrimidin und Piperidin-4-carbonsäureäthylester.
Schmelzpunkt: 192–194 °C (Äthanol).

Beispiel 40
2-(3-Äthoxycarbonyl-piperidino)8-benzylthio -4-(1-oxido-thiomorpholino)- pyrimido[5,4-d]-pyrimidin
Hergestellt analog Beispiel 1 aus 8-Benzylthio-2-chlor -4- (1-oxido-thiomorpholino)- pyrimido[5,4-d]pyrimidin und Piperidin-3-carbonsäureäthylester.
Schmelzpunkt: 167–170 °C (Äthanol).

Beispiel 41
8-Benzylthio-2-morpholino-4-(1-oxido-thiomorpholino)pyrimido[5,4-d]pyrimidin
Hergestellt analog Beispiel 1 aus 8-Benzylthio-2-chlor-4- (1-oxido-thiomorpholino)- pyrimido-[5,4-d]pyrimidin- und Morpholin.
Schmelzpunkt: 239–240 °C (Äthanol).

Beispiel 42
8-Benzylthio-4-morpholino-2-(1-oxido-thiomorpholino)pyrimido[5,4-d]pyrimidin
Hergestellt analog Beispiel 1 aus 8-Benzylthio-2-chlor-4-morpholino-pyrimido [5,4-d] pyrimidin (Schmelzpunkt: 159–161 °C) und Thiomorpholin-1-oxid.
Schmelzpunkt: 245–247 °C.

Beispiel 43
8-Benzylthio-2-(1-oxido-thiomorpholino)-4-thiomorpholino-pyrimido[5,4- d]pyrimidin
Hergestellt analog Beispiel 1 aus 8-Benzylthio-2-chlor -4- thiomorpholino-pyrimido [5,4-d]pyrimidin (Schmelzpunkt: 175–177 °C) und Thiomorpholin-1-oxid.
Schmelzpunkt: 204–205 °C.

Beispiel 44
8-Benzylthio-2,4-bis- (1-oxido-thiomorpholino)pyrimido[5,4-d]-pyrimidin
Hergestellt analog Beispiel 1 aus 8-Benzylthio-2-chlor -4- (1-oxido-thiomorpholino)- pyrimido[5,4-d]pyrimidin (Schmelzpunkt: 188–190 °C) und Thiomorpholin-1-oxid durch dreistündiges Erhitzen in Dioxan unter Rückfluss.
Schmelzpunkt: 208–211 °C.

Beispiel 45
8-Benzylthio-2-imidazolyl -4- (1-oxido-thio-

morpholino)-pyrimido-[5,4-d]pyrimidin

Hergestellt analog Beispiel 1 aus 8-Benzylthio-2-chlor-4-(1-oxido-thiomorpholino)-pyrimido[5,4-d]pyrimidin und Imidazol durch sechsstündiges Erhitzen in Dioxan unter Rückfluss.

Schmelzpunkt: 260–262 °C.

Beispiel 46

2-(2-Hydroxyäthylamino)-4-(1-oxido-thiomorpholino)-8-phenäthylthio-pyrimido[5,4-d]-pyrimidin

Hergestellt analog Beispiel 1 aus 2-Chlor-4-(1-oxido-thiomorpholino)-8-phenäthylthio-pyrimido[5,4-d]pyrimidin (Schmelzpunkt: 248–250 °C) und 2-Hydroxyäthylamin.

Schmelzpunkt: 181–184 °C (Äthanol).

Beispiel 47

2-Diäthanolamin-4-(1-oxido-thiomorpholino)-8-phenäthylthio-pyrimido[5,4-d]pyrimidin

Hergestellt analog Beispiel 1 aus 2-Chlor-4-(1-oxido-thiomorpholino)-8-phenäthylthio-pyrimido[5,4-d] pyrimidin und Diäthanolamin.

Schmelzpunkt: 178–179 °C (Äthanol).

Beispiel 48

2-(2-Hydroxyäthylamino)-4-(1-oxido-thiomorpholino)-8-(3-phenylpropylthio)-pyrimido-[5,4-d]pyrimidin

Hergestellt analog Beispiel 1 aus 2-Chlor-4-(1-oxido-thiomorpholino)-8-(3-phenylpropylthio)-pyrimido[5,4-d]pyrimidin (Schmelzpunkt: 172–174 °C) und 2-Hydroxyäthylamin.

Schmelzpunkt: 180–182 °C (Äthanol).

Beispiel 49

8-Furfurylthio-2-(2-hydroxyäthylamino)-4-(1-oxido-thiomorpholino)-pyrimido[5,4-d]pyrimidin

Hergestellt analog Beispiel 1 aus 2-Chlor-8-furfurylthio-4-(1-oxido-thiomorpholino)-pyrimido[5,4-d]pyrimidin (Schmelzpunkt: 173–175 °C) und 2-Hydroxyäthylamin.

Schmelzpunkt: 182–184 °C (Äthanol).

Beispiel 50

2-(2-Hydroxyäthylamino)-8-(2-hydroxyäthylthio)-4-(1-oxido-thiomorpholino)-pyrimido-[5,4-d]pyrimidin

Hergestellt analog Beispiel 1 aus 2-Chlor-8-(2-hydroxyäthylthio)-4-(1-oxido-thiomorpholino)-pyrimido[5,4-d]pyrimidin (Schmelzpunkt: 228–230 °C, Zers.) und 2-Hydroxyäthylamin.

Schmelzpunkt: 200–203 °C (Äthanol/Essigsäureäthylester).

Beispiel 51

2-Diäthanolamin-8-(2-hydroxyäthylthio)-4-(1-oxido-thiomorpholino)-pyrimido[5,4-d]pyrimidin

Hergestellt analog Beispiel 1 aus 2-Chlor-8-(2-hydroxyäthylthio)-4-(1-oxido-thiomorpholino)-pyrimido[5,4-d]pyrimidin und Diäthanolamin.

Schmelzpunkt: 196–199 °C (Äthanol).

Beispiel 52

8-Äthoxycarbonylmethylthio-2-(2-hydroxyäthylamino)-4-1-oxido-thiomorpholino)-pyrimido[5,4-d]pyrimidin

Hergestellt analog Beispiel 1 aus 8-Äthoxycarbonylmethylthio-2-chlor-4-(1-oxido-thiomorpholino)-pyrimido[5,4-d]pyrimidin (Schmelzpunkt: 174–175 °C und 2-Hydroxyäthylamin.

Schmelzpunkt: 172–174 °C (Methanol).

Beispiel 53

8-(2-Diäthylaminoäthylthio)-2-(2-hydroxypropylamino)-4-(1-oxido-thiomorpholino)-pyrimido[5,4-d]pyrimidin

Hergestellt analog Beispiel 1 aus 2-Chlor-8-(2-diäthylaminoäthylthio)-4-(1-oxido-thiomorpholino)-pyrimido[5,4-d]pyrimidin (Schmelzpunkt: 146–148 °C) und 2-Hydroxypropylamin.

Harz.

Beispiel 54

2-(2-Hydroxyäthylamino)-8-(2-indanylthio)-4-(1-oxido-thiomorpholino)-pyrimidino-[5,4-d]pyrimidino

Hergestellt analog Beispiel 1 aus 2-Chlor-8-(2-indanylthio)-4-(1-oxido-thiomorpholino)-pyrimido[5,4-d]pyrimidin (Schmelzpunkt: 268–270 °C) und 2-Hydroxyäthylamin.

Schmelzpunkt: 228–230 °C (Dimethylformamid).

Beispiel 55

2-(2-Hydroxyäthylamino)-8-(1-naphthyl methylthio)-4(1-oxido-thiomorpholino)-pyrimido[5,4-d]pyrimidin

Hergestellt analog Beispiel 1 aus 2-Chlor-8-(1-naphthylmethylthio)-4-(1-oxido-thiomorpholino)-pyrimido[5,4-d]pyrimidin (Schmelzpunkt: 212–215 °C) und 2-Hydroxyäthylamin.

Schmelzpunkt: 210–211 °C (Dimethylformamid).

Beispiel 56

2-(2-Hydroxyäthylamino)-4-(1-oxido-thiomorpholino)-8-phenylthiopyrimido[5,4-d]pyrimidin

Hergestellt analog Beispiel 1 aus 2-Chlor-4-(1-oxido-thiomorpholino)-8-phenylthio-pyrimido[5,4-d]pyrimidin (Schmelzpunkt: 253–255°C) und 2-Hydroxyäthylamin.

Schmelzpunkt: 242–245 °C.

Beispiel 57

2-(2-Hydroxyäthylamino)-4-(1-oxido-thiomorpholino)-8-(4-tolylthio)-pyrimido[5,4-d]-pyrimidin

Hergestellt analog Beispiel 1 aus 2-Chlor-4-(1-oxido-thiomorpholino)-8-(4-tolylthio)-pyrimido[5,4-d]pyrimidin (Schmelzpunkt: 261–263°C) und 2-Hydroxyäthylamin.

Schmelzpunkt: 245–247 °C (Äthanol/Essigsäureäthylester).

Beispiel 58

2-(2-Hydroxyäthylamino) -8- (4-methoxyphenylthio) -4- (1-oxido-thiomorpholino)- pyrimido[5,4-d]pyrimidin

Hergestellt analog Beispiel 1 aus 2-Chlor-8-(4-methoxyphenylthio) -4- (1-oxido-thiomorpholino) -pyrimido[5,4-d]pyrimidin (Schmelzpunkt: 239–241 °C (und 2-Hydroxyäthylamin.

Schmelzpunkt: 221–223 °C (Methanol/Dioxan).

Beispiel 59

2-Diäthanolamino -8- (4-hydroxyphenylthio) - 4- (1-oxido-thiomorpholino)- pyrimido[5,4-d] pyrimidin

Hergestellt analog Beispiel 1 aus 2-Chlor -8-(4-hydroxyphenylthio) -4- (1-oxido-thiomorpholino)- pyrimido[5,4-d]pyrimidin (Schmelzpunkt: > 280 °C) und Diäthanolamin.

Schmelzpunkt: 223–225 °C (Äthanol).

Beispiel 60

2-Diäthanolamino -8- (4-fluorphenylthio) -4-(1-oxido-thiomorpholino)- pyrimido[5,4-d]pyrimidin

Hergestellt analog Beispiel 1 aus 2-Chlor -8-(4-fluorphenylthio) -4- (1-oxido-thiomorpholino) -pyrimido[5,4-d]pyrimidin (Schmelzpunkt: 243–245 °C) und Diäthanolamin.

Schmelzpunkt: 210–212 °C (Essigsäureäthylester/Methanol).

Beispiel 61

8-(4-Chlorphenylthio)- 2-(2-hydroxyäthylamino) -4- (1-oxido-thiomorpholino)-pyrimido[5,4-d]pyrimidin

Hergestellt analog Beispiel 1 aus 2-Chlor -8-(4-chlorphenylthio) -4- (1-oxido-thiomorpholino)- pyrimido[5,4-d]pyrimidin (Schmelzpunkt: 267–269 °C) und 2-Hydroxyäthylamin.

Schmelzpunkt: 228–230 °C (Methanol).

Beispiel 62

8-(4-Bromphenylthio) -2- diäthanolamino -4-(1-oxido-thiomorpholino)- pyrimido[5,4-d]pyrimidin

Hergestellt analog Beispiel 1 aus 8-(4-Bromphenylthio) -2- chlor -4- (1-oxido-thiomorpholino)- pyrimido[5,4-d]pyrimidin (Schmelzpunkt: 272–275 °C) und Diäthanolamin.

Schmelzpunkt: 176–178 °C (Essigsäureäthylester/Methanol).

Beispiel 63

8-Benzylthio -4- (2,6-dimethylmorpholino) -2-(2-hydroxyäthylamino)- pyrimido[5,4-d]pyrimidin

Hergestellt analog Beispiel 1 aus 8-Benzylthio-2-chlor -4- (2,6-dimethylmorpholino)- pyrimido[5,4-d]pyrimidin (Schmelzpunkt: 85–90 °C) und 2-Hydroxyäthylamin.

Schmelzpunkt: 96–99 °C (Äthanol).

Beispiel 64

8-Amino -2- (2-hydroxyäthylamino) -4- (1-oxido-thiomorpholino)- pyrimido[5,4-d]pyrimidin

Hergestellt analog Beispiel 2 aus 8-Amino -2-chlor -4- (1-oxido-thiomorpholino)- pyrimido[5,4-d]pyrimidin (Schmelzpunkt: 270–272 °C; Zers.) und 2-Hydroxyäthylamin.

Schmelzpunkt: 221–223 °C (Umfällung aus 0,1 n-Salzsäure mittels Ammoniak).

Beispiel 65

2-(2-Hydroxyäthylamino) -8-methylamino -4-(1-oxido-thiomorpholino)- pyrimido[5,4-d]pyrimidin

Hergestellt analog Beispiel 2 aus 2-Chlor -8-methylamino -4- (1-oxido-thiomorpholino)pyrimido[5,4-d] pyrimidin (Schmelzpunkt: 278–280 °C) und 2-Hydroxyäthylamin.

Schmelzpunkt: 175–177 °C (Dioxan).

Beispiel 66

2-(2-Hydroxyäthylamino) -4- (1-oxido-thiomorpholino)- 8-propylaminopyrimido[5,4-d]pyrimidin

Hergestellt analog Beispiel 2 aus 2-Chlor -4-(1-oxido-thiomorpholino)- 8-propylamino-pyrimido[5,4-d]pyrimidin (Schmelzpunkt: 174–176 °C) und 2-Hydroxyäthylamin.

Schmelzpunkt: 164–166 °C (Methanol).

Beispiel 67

2-(2-Hydroxyäthylamino) -8-isopropylamino - 4- (1-oxido-thiomorpholino)- pyrimido[5,4-d] pyrimidin

Hergestellt analog Beispiel 2 aus 2-Chlor -8-isopropylamino -4- (1-oxido-thiomorpholino)-pyrimido[5,4-d]pyrimidin (Schmelzpunkt: 213–215 °C) und 2-Hydroxyäthylamin.

Schmelzpunkt: 154–156 °C (Essigsäureäthylester).

Beispiel 68

8-Butylamino -2- (2-hydroxyäthylamino) -4- (1-oxido-thiomorpholino)-pyrimido[5,4-d]pyrimidin

Hergestellt analog Beispiel 2 aus 8-Butylamino -2- chlor -4- (1-oxido-thiomorpholino)-pyrimido [5,4-d]pyrimidin (Schmelzpunkt: 179–181 °C) und 2-Hydroxyäthylamin.

Schmelzpunkt: 157–159 °C (Methanol).

Beispiel 69

2-(2-Hydroxyäthylamino) -8-isopentylamino -4-(1-oxido-thiomorpholino)-pyrimido[5,4-d]pyrimidin

Hergestellt analog Beispiel 2 aus 2-Chlor -8-isopentylamino -4- (1-oxido-thiomorpholino)-pyrimido[5,4-d]pyrimidin (Schmelzpunkt: 176–178 °C) und 2-Hydroxyäthylamin.

Schmelzpunkt: 173–174 °C (Essigsäureäthylester).

Beispiel 70

2-(2-Hydroxyäthylamino) -8-octylamino -4- (1-

oxido-thiomorpholino)- pyrimido[5,4-d]pyrimidin

Hergestellt analog Beispiel 2 aus 2-Chlor-8-octylamino-4-(1-oxido-thiomorpholino)-pyrimido [5,4-d]pyrimidin (Schmelzpunkt: 145–147 °C) und 2-Hydroxyäthylamin.

Schmelzpunkt: 137–139 °C (Methanol).

Beispiel 71

8-(N-Äthyl-butylamino)-2-(2-hydroxyäthylamino)-4-(1-oxido-thiomorpholino)-pyrimido [5,4-d]pyrimidin

Hergestellt analog Beispiel 2 aus 8-(N-Äthylbutylamino)-2-chlor-4-(1-oxido-thiomorpholino)-pyrimido[5,4-d]pyrimidin (Schmelzpunkt: 157–158 °C) und 2-Hydroxyäthylamin.

Schmelzpunkt: 154–155 °C (Methanol).

Beispiel 72

8-Dibutylamino-2-(2-hydroxyäthylamino)-4(1-oxido-thiomorpholino)-pyrimido[5,4-d] pyrimidin

Hergestellt analog Beispiel 2 aus 2-Chlor-8-dibutylamino-4-(1-oxido-thiomorpholino)-pyrimido[5,4-d]pyrimidin (Schmelzpunkt: 187–189 °C) und 2-Hydroxyäthylamin.

Schmelzpunkt: 175–177 °C (Methanol).

Beispiel 73

8-(N-Cyclohexyl-methylamino)-2-(2-hydroxyäthylamino)-4-(1-oxido-thiomorpholino)-pyrimido[5,4-d]pyrimidin

Hergestellt analog Beispiel 2 aus 2-Chlor-8-(N-cyclohexylmethylamino)-4-(1-oxido-thiomorpholino)-pyrimido [5,4-d] pyrimidin (Schmelzpunkt: 226–228 °C) und 2-Hydroxyäthylamin.

Schmelzpunkt: 222–223 °C (Methanol).

Beispiel 74

8-Cyclohexylmethylamino-2-(2-hydroxyäthylamino)-4-(1-oxido-thiomorpholino)-pyrimido[5,4-d]pyrimidin

Hergestellt analog Beispiel 2 aus 2-Chlor-8-cyclohexylmethylamino-4-(1-oxido-thiomorpholino)-pyrimido[5,4-d]pyrimidin (Schmelzpunkt: 217–218 °C) und 2-Hydroxyäthylamin.

Schmelzpunkt: 155–156 °C (Methanol).

Beispiel 75

8-Benzylamino-2-(2-hydroxyäthylamino)-4-(1-oxido-thiomorpholino)-pyrimido[5,4-d]pyrimidin

Hergestellt analog Beispiel 2 aus 8-Benzylamino-2-chlor-4-(1-oxido-thiomorpholino)-pyrimido[5,4-d]pyrimidin (Schmelzpunkt: 232–233 °C) und 2-Hydroxyäthylamin.

Schmelzpunkt: 192–194 °C (Äthanol/Dioxan).

Schmelzpunkt des Maleinats: 170–175 °C.

Beispiel 76

8-Benzylamino-2-(2-hydroxyäthylamino)-4-morpholino-pyrimido[5,4-d] pyrimidin

Hergestellt analog Beispiel 2 aus 8-Benzylamino-2-chlor-4-morpholino-pyrimido [5,4-d]pyrimidin (Schmelzpunkt: 139–141 °C) und 2-Hydroxyäthylamin.

Schmelzpunkt: 130–132 °C.

Beispiel 77

8-Benzylamino-2-(2-hydroxyäthylamino)-4-thiomorpholino-pyrimido-[5,4-d]pyrimidin

Hergestellt analog Beispiel 2 aus 8-Benzylamino-2-chlor-4-thiomorpholino-pyrimido [5,4-d] pyrimidin (Schmelzpunkt: 129–130 °C) und 2-Hydroxyäthylamin.

Schmelzpunkt: 109–111 °C.

Beispiel 78

8-Benzylamino-2-(2-hydroxyäthylamino)-4-(1,1-dioxido-thiomorpholino)-pyrimido[5,4-d] pyrimidin

Hergestellt analog Beispiel 2 aus 8-Benzylamino-2-chlor-4-(1,1-dioxido-thiomorpholino)-pyrimido[5,4-d]pyrimidin (Schmelzpunkt: 213–215 °C) und 2-Hydroxyäthylamin.

Schmelzpunkt: 204–205 °C.

Die Substanz wird auch aus 8-Benzylamino-2-(2-hydroxyäthylamino)-4-thiomorpholino-pyrimido[5,4-d]pyrimidin durch Oxidation mit Kaliumpermanganat in verdünnter Salzsäure erhalten.

Beispiel 79

8-Benzylamino-2-diäthanolamino-4-(1-oxido-thiomorpholino)-pyrimido[5,4-d]pyrimidin

Hergestellt analog Beispiel 3 aus 8-Benzylamino-2-chlor-4-(1-oxido-thiomorpholino)-pyrimido[5,4-d]pyrimidin (Schmelzpunkt: 232–233 °C) und Diäthanolamin.

Schmelzpunkt: 195–196 °C (Methanol).

Beispiel 80

8-Benzylamino-2-[N-(2-hydroxyäthyl)-2-methoxyäthylamino]-4-(1-oxido-thiomorpholino)-pyrimido[5,4-d]pyrimidin

Hergestellt analog Beispiel 3 aus 8-Benzylamino-2-chlor-4-(1-oxido-thiomorpholino)-pyrimido[5,4-d]pyrimidin und N-(2-Hydroxyäthyl-2-methoxyäthylamin.

Schmelzpunkt: 145–147 °C (Methanol).

Beispiel 81

2-Diäthanolamino-4-(1-oxido-thiomorpholino)-8-phenäthylamino-pyrimido[5,4-d]pyrimidin

Hergestellt analog Beispiel 3 aus 2-Chlor-4-(1-oxido-thiomorpholino)-8-phenäthylamino-pyrimido [5,4-d]pyrimidin (Schmelzpunkt: 202–204 °C) und Diäthanolamin.

Schmelzpunkt: 194–196 °C (Methanol).

Beispiel 82

2-[N-(2-Hydroxyäthyl)-2-methoxyäthylamino]-4-(1-oxido-thiomorpholino)-8-phenäthylamino-pyrimido[5,4-d]pyrimidin

Hergestellt analog Beispiel 3 aus 2-Chlor-4-(1-oxido-thiomorpholino)-8-phenäthylamino-

pyrimido[5,4-d]pyrimidin und N-(2-Hydroxyäthyl) -2- methoxyäthylamin.

Schmelzpunkt: 115–117 °C (Methanol/Wasser).

Beispiel 83

2-Diäthanolamino -4- (1-oxido-thiomorpholino)-8-(3-phenylpropylamino)-pyrimido-[5,4-d]pyrimidin

Hergestellt analog Beispiel 3 aus 2-Chlor -4-(1-oxido-thiomorpholino)- 8- (3-phenylpropylamino)- pyrimido[5,4-d]pyrimidin (Schmelzpunkt: 152–154 °C) und Diäthanolamin.

Schmelzpunkt: 164–166 °C (Essigsäureäthylester/ Methanol).

Beispiel 84

2-(2-Hydroxyäthylamino)- 4-(1-oxido-thiomorpholino)- 8-phenäthylamino-pyrimido [5,4-d] pyrimidin

Hergestellt analog Beispiel 2 aus 2-Chlor -4-(1-oxido-thiomorpholino)- 8-phenäthylamino-pyrimido[5,4-d]pyrimidin (Schmelzpunkt: 202–204 °C) und 2-Hydroxyäthylamin.

Schmelzpunkt: 187–189 °C (Methanol).

Schmelzpunkt des Hydrochlorids: 161–164 °C.

Schmelzpunkt des Maleinats: 156–158 °C.

Beispiel 85

2-(2-Hydroxyäthylamino)-4-(1-oxido-thiomorpholino) -8- (3-phenylpropylamino)- pyrimido [5,4-d]pyrimidin

Hergestellt analog Beispiel 2 aus 2-Chlor-4-(1-oxido-thiomorpholino) -8- (3-phenylpropylamino) -pyrimido[5,4-d]pyrimidin (Schmelzpunkt: 152–154 °C) und 2-Hydroxyäthylamin.

Schmelzpunkt: 164–166 °C (Methanol).

Beispiel 86

2-(2-Hydroxyäthylamino)-4- (1-oxido-thiomorpholino)- 8-(4-phenylbutylamino)- pyrimido [5,4-d]pyrimidin

Hergestellt analog Beispiel 2 aus 2-Chlor-4-(1-oxido-thiomorpholino) -8- (4-phenylbutylamino)- pyrimido[5,4-d]pyrimidin (Schmelzpunkt: 156–158 °C) und 2-Hydroxyäthylamin.

Schmelzpunkt: 164–166 °C (Essigsäureäthylester).

Beispiel 87

2-(2-Hydroxyäthylamino) -4- (1-oxido-thiomorpholino) -8(L-1-phenyl-äthylamino)- pyrimido [5,4-d]pyrimidin

Hergestellt analog Beispiel 2 aus 2-Chlor-4-(1-oxido-thiomorpholino) -8- (L-1-phenyläthylamino)- pyrimido[5,4-d]pyrimidin (Schmelzpunkt: 167–169 °C) und 2-Hydroxyäthylamin.

Schmelzpunkt: 85–88 °C.

Beispiel 88

8-Benzylamino-2- (5-hydroxypentylamino) -4-(1-oxido-thiomorpholino)- pyrimido[5,4-d]pyrimidin

Hergestellt analog Beispiel 2 aus 8-Benzylamino-2-chlor -4- (1-oxido-thiomorpholino)- pyrimido [5,4-d]pyrimidin (Schmelzpunkt: 232–233 °C) und 5-Amino-1-pentanol.

Schmelzpunkt: 132–134 °C (Methanol).

Beispiel 89

8-Benzylamino-2- (5-hydroxy-1,5-dimethylhexylamino) -4- (1-oxido-thiomorpholino)- pyrimido[5,4-d]pyrimidin

Hergestellt analog Beispiel 3 aus 8-Benzylamino-2-chlor-4- (1-oxido-thiomorpholino)- pyrimido[5,4-d]pyrimidin (Schmelzpunkt: 232–233 °C) und 6-Amino-2-methyl-2-heptanol. Harz.

Beispiel 90

8-Benzylamino-2- (3-hydroxypiperidino)- 4-(1-oxido-thiomorpholino)- pyrimido[5,4-d] pyrimidin

Hergestellt analog Beispiel 2 aus 8-Benzylamino-2-chlor-4- (1-oxido-thiomorpholino)- pyrimido[5,4-d]pyrimidin (Schmelzpunkt: 232–233 °C) und 3-Hydroxy-piperidin.

Schmelzpunkt: 213–215 °C (Äthanol).

Beispiel 91

8-(N-Benzyl-methylamino)- 2-diäthanolamino-4-(1-oxido-thiomorpholino)-pyrimido[5,4-d]-pyrimidin

Hergestellt analog Beispiel 3 aus 8-(N-Benzyl-methylamino)-2-chlor-4- (1-oxido-thiomorpholino)- pyrimido[5,4-d]pyrimidin (Schmelzpunkt: 158–160 °C) und Diäthanolamin.

Schmelzpunkt: 159–161 °C (Methanol).

Beispiel 92

8-(N-Benzyl-methylamino)-2- (2-hydroxyäthylamino)- 4-thiomorpholino-pyrimido[5,4-d] pyrimidin

Hergestellt analog Beispiel 2 aus 8-(N-Benzyl-methylamino) -2-chlor-4-thiomorpholino-pyrimido [5,4-d] pyrimidin (Schmelzpunkt: 95–97 °C) und 2-Hydroxyäthylamin.

Schmelzpunkt: 112–114 °C.

Beispiel 93

8-(N-Benzyl-methylamino) -2- (2-hydroxyäthylamino) -4-morpholino- pyrimido[5,4-d]pyrimidin

Hergestellt analog Beispiel 2 aus 8-(N-Benzyl-methylamino) -2-chlor-4-morpholino-pyrimido [5,4-d]pyrimidin (Schmelzpunkt: 121–123 °C) und 2-Hydroxyäthylamin.

Schmelzpunkt: 106–108 °C (Methanol).

Beispiel 94

8-(N-Äthyl-benzylamino) -2-(2-hydroxyäthylamino) -4- (1-oxido-thiomorpholino)- pyrimido [5,4-d]pyrimidin

Hergestellt analog Beispiel 2 aus 8-(N-Äthylbenzylamino) -2-chlor-4- (1-oxido-thiomorpholino) -pyrimido[5,4-d]pyrimidin (Schmelzpunkt: 163–165 °C) und 2-Hydroxyäthylamin.

Schmelzpunkt: 164–165 °C (Methanol).

Beispiel 95

8-(N-Benzyl-propylamino-2- (2-hydroxyäthylamino) -4- (1-oxido-thiomorpholino)- pyrimido [5,4-d]pyrimidin

Hergestellt analog Beispiel 2 aus 8-(N-Benzylpropylamino)2-chlor-4- (1-oxido-thiomorpholino)- pyrimido[5,4-d]pyrimidin (Schmelzpunkt: 183–184 °C) und 2-Hydroxyäthylamin.

Schmelzpunkt: 167–169 °C (Essigsäureäthylester).

Beispiel 96

8-(N-Benzyl-butylamino)- 2-(2-hydroxyäthylamino) -4(1-oxido-thiomorpholino)- pyrimido [5,4-d]pyrimidin

Hergestellt analog Beispiel 2 aus 8-(N-Benzylbutylamino)- 2-chlor-4- (1-oxido-thiomorpholino)- pyrimido[5,4-d]pyrimidin (Schmelzpunkt: 153–155 °C) und 2-Hydroxyäthylamin.

Schmelzpunkt: 162–163 °C (Umfällung aus 2 n-Salzsäure mittels Ammoniak).

Beispiel 97

8-[N-Benzyl- (2-hydroxyäthylamino)]-2- (2-hydroxyäthylamino)- 4-(1-oxido-thiomorpholino) -pyrimido[5,4-d]pyrimidin

Hergestellt analog Beispiel 2 aus 8-[N-Benzyl-(2-hydroxyäthylamino)]- 2-chlor-4- (1-oxido-thiomorpholino) -pyrimido[5,4-d]pyrimidin (Schmelzpunkt: 153–155 °C) und 2-Hydroxyäthylamin.

Schmelzpunkt: 140–142 °C (Essigsäureäthylester/Methanol).

Beispiel 98

2,8-Bis(2-hydroxyäthylamino) -4- (1-oxido-thiomorpholino)- pyrimido[5,4-d]pyrimidin

Hergestellt analog Beispiel 2 aus 2,8-Dichlor-4- (1-oxido-thiomorpholino)- pyrimido[5,4-d]pyrimidin (Schmelzpunkt: 195–198 °C) und 2-Hydroxyäthylamin.

Schmelzpunkt: 156–158 °C (Wasser).

Beispiel 99

2-(2-Hydroxyäthylamino) -8- (5-hydroxypentylamino) -4- (1-oxido-thiomorpholino)- pyrimido[5,4-d]pyrimidin

Hergestellt analog Beispiel 2 aus 2-Chlor-8-(5-hydroxypentylamino) -4- (1-oxido-thiomorpholino)- pyrimido[5,4-d]pyrimidin (Schmelzpunkt: 202–204 °C) und 2-Hydroxyäthylamin.

Schmelzpunkt: 121–123 °C (Umfällung aus 0,1 n-Salzsäure mittels Ammoniak).

Beispiel 100

2-(2-Hydroxyäthylamino) -8- (5-hydroxy-1,5-dimethylhexylamino) -4- (1-oxido-thiomorpholino) -pyrimido[5,4-d]pyrimidin

Hergestellt analog Beispiel 2 aus 2-Chlor-8-(5-hydroxy-1,5-dimethylhexylamino) -4- (1-oxido-thiomorpholino)- pyrimido[5,4-d]pyrimidin (Harz) und 2-Hydroxyäthylamin. Harz.

Beispiel 101

8-(3-Äthoxypropylamino) -2- (2-hydroxyäthylamino) -4- (1-oxido-thiomorpholino)- pyrimido[5,4-d] pyrimidin

Hergestellt analog Beispiel 2 aus 8-(3-Äthoxypropylamino) -2-chlor-4(1-oxido-thiomorpholino)- pyrimido[5,4-d]pyrimidin (Schmelzpunkt: 153–154 °C) und 2-Hydroxyäthylamin.

Schmelzpunkt: 149–151 °C (Essigsäureäthylester).

Beispiel 102

8-Furfurylamino-2- (2-hydroxyäthylamino) -4-(1-oxido-thiomorpholino)- pyrimido[5,4-d]pyrimidin

Hergestellt analog Beispiel 2 aus 2-Chlor-8-furfurylamino-4- (1-oxido-thiomorpholino)pyrimido[5,4-d]pyrimidin (Schmelzpunkt: 203–205 °C) und 2-Hydroxyäthylamin.

Schmelzpunkt: 194–196 °C (Methanol).

Beispiel 103

2-(2-Hydroxypropylamino) -4- (1-oxido-thiomorpholino) -8- (3-picolylamino)- pyrimido[5,4-d]pyrimidin

Hergestellt analog Beispiel 2 aus 2-Chlor-4-(1-oxido-thiomorpholino) -8- (3-picolylamino)-pyrimido[5,4-d]pyrimidin (Schmelzpunkt: 227–229 °C) und 1-Amino-2-propanol.

Schmelzpunkt: 197–199 °C (Umfällung aus 0,1 n-Salzsäure mittels Ammoniak).

Beispiel 104

2-(2-Hydroxyäthylamino)-8- [N-methyl- (3-picolylamino)]- 4-(1-oxido-thiomorpholino)- pyrimido[5,4-d]pyrimidin

Hergestellt analog Beispiel 2 aus 2-Chlor-8-[N-methyl- (3-picolylamino)]- 4-(1-oxido-thiomorpholino)pyrimido[5,4-d]pyrimidin (Schmelzpunkt: 154–156 °C) und 2-Hydroxyäthylamin.

Schmelzpunkt: 186–188 °C(Umfällung aus 0,1 n-Salzsäure mittels Ammoniak).

Beispiel 105

2-(2-Hydroxyäthylamino) -8- (N-methylanilino) -4-(1-oxido-thiomorpholino)-pyrimido[5,4-d]-pyrimidin

Hergestellt analog Beispiel 2 aus 2-Chlor-8-(N-methylanilino) -4- (1-oxido-thiomorpholino)- pyrimido[5,4-d]pyrimidin (Schmelzpunkt: 237–239 °C) und 2-Hydroxyäthylamin.

Schmelzpunkt: 217–219 °C (Umfällung aus 0,1 n-Salzsäure mittels Ammoniak).

Beispiel 106

8-(4-Äthoxyanilino) -2- (2-hydroxyäthylamino) -4- (1-oxido-thiomorpholino)- pyrimido [5,4-d] pyrimidin

Hergestellt analog Beispiel 3 aus 8-(4-Äthoxyanilino) -2-chlor-4- (1-oxido-thiomorpholino)-pyrimido[5,4-d]pyrimidin (Schmelzpunkt: 237–240 °C) und 2-Hydroxyäthylamin.

Schmelzpunkt: 219–221 °C.

Beispiel 107

2-(2-Hydroxyäthylamino)-4-(1-oxido-thiomorpholino)-8-(3-trifluormethylanilino)-pyrimido[5,4-d]pyrimidin

Hergestellt analog Beispiel 3 aus 2-Chlor-4-(1-oxido-thiomorpholino)-8-(3-trifluormethylanilino)-pyrimido[5,4-d]pyrimidin (Schmelzpunkt: 215–218 °C) und 2-Hydroxyäthylamin.

Schmelzpunkt: 233–235 °C (Äthanol).

Beispiel 108

2-(2-Hydroxyäthylamino)-8-(4-methylbenzylamino)-4-(1-oxido-thiomorpholino)-pyrimido[5,4-d]pyrimidin

Hergestellt analog Beispiel 2 aus 2-Chlor-8-(4-methylbenzylamino)-4-(1-oxido-thiomorpholino)-pyrimido[5,4-d]pyrimidin (Schmelzpunkt: 185–187 °C) und 2-Hydroxyäthylamin.

Schmelzpunkt: 173–175 °C (Essigsäureäthylester).

Beispiel 109

2-(2-Hydroxyäthylamino)-8-(3-methoxybenzylamino)-4-(1-oxido-thiomorpholino)-pyrimido[5,4-d]pyrimidin

Hergestellt analog Beispiel 2 aus 2-Chlor-8-(3-methoxybenzylamino)-4 (1-oxido-thiomorpholino)-pyrimido[5,4-d)pyrimidin (Schmelzpunkt: 222–224 °C) und 2-Hydroxyäthylamin.

Schmelzpunkt: 168–170 °C (Essigsäureäthylester/Dioxan).

Beispiel 110

2-(2-Hydroxyäthylamino)-8-(4-methoxybenzylamino)-4(1-oxido-thiomorpholino)pyrimido[5,4-d]pyrimidin

Hergestellt analog Beispiel 2 aus 2-Chlor-8-(4-methoxybenzylamino)-4-(1-oxido-thiomorpholino)-pyrimido[5,4-d]pyrimidin (Schmelzpunkt: 220–222 °C) und 2-Hydroxyäthylamin.

Schmelzpunkt: 173–175 °C (Essigsäureäthylester/Dioxan).

Beispiel 111

8-(3,4-Dimethoxybenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(3,4-dimethoxybenzylamino)-4-(1-oxido-thiomorpholino)-pyrimido[5,4-d]pyrimidin (Schmelzpunkt: 218–220 °C) und 2-Hydroxyäthylamin.

Schmelzpunkt: 175–177 °C (Methanol).

Beispiel 112

2-(2-Hydroxyäthylamino)-8-(3,4-methylendioxy-benzylamino)-4-(1-oxido-thiomorpholino)-pyrimido[5,4-d]pyrimidin

Hergestellt analog Beispiel 2 aus 2-Chlor-8-(3,4-methylendioxybenzylamino)-4-(1-oxido-thiomorpholino)-pyrimido[5,4-d]pyrimidin (Schmelzpunkt: 239–241 °C) und 2-Hydroxyäthylamin.

(Schmelzpunkt: 216–218 °C (Dioxan).

Beispiel 113

8-(4-Fluorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(4-fluorbenzylamino)-4-(1-oxido-thiomorpholino)-pyrimido[5,4-d]pyrimidin (Schmelzpunkt: 210–212 °C) und 2-Hydroxyäthylamin.

Schmelzpunkt: 190–192 °C (Essigsäureäthylester/Methanol).

Beispiel 114

8-(2-Chlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(2-chlorbenzylamino)-4(1-oxido-thiomorpholino)-pyrimido[5,4-d]pyrimidin (Schmelzpunkt: 238–240 °C) und 2-Hydroxyäthylamin.

Schmelzpunkt: 178–180 °C (Methanol).

Beispiel 115

8-(3-Chlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(3-chlorbenzylamino)-4-(1-oxido-thiomorpholino)-pyrimido[5,4-d]pyrimidin (Schmelzpunkt: 239–241 °C) und 2-Hydroxyäthylamin.

Schmelzpunkt: 203–204 °C (Dioxan).

Beispiel 116

8-(4-Chlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-chlorbenzylamino)-4-(1-oxido-thiomorpholino)-pyrimido[5,4-d]pyrimidin (Schmelzpunkt: 216–218 °C) und 2-Hydroxyäthylamin.

Schmelzpunkt: 215–217 °C (Dioxan).

Beispiel 117

8-(2,4-Dichlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(2,4-dichlorbenzylamino)-4-(1-oxido-thiomorpholino)-pyrimido[5,4-d]pyrimidin (Schmelzpunkt: 196–198 °C) und 2-Hydroxyäthylamin.

Schmelzpunkt: 210–212 °C (Dioxan).

Beispiel 118

8-(3,4-Dichlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(3,4-dichlorbenzylamino)-4-(1-oxido-thiomorpholino)-pyrimido[5,4-d] pyrimidin (Schmelzpunkt: 259–261 °C) und 2-Hydroxyäthylamin.

Schmelzpunkt: 186–188 °C (Dioxan).

Beispiel 119
8-(3,4-Dimethoxyphenäthylamino)-2-(2-hydroxyäthylamino)-4-(1-oxido-thiomorpholino)-pyrimido[5,4-d]pyrimidin

Hergestellt analog Beispiel 2 aus 2-Chlor-8-(3,4-dimethoxyphenäthylamino)-4-(1-oxido-thiomorpholino)-pyrimido[5,4-d]pyrimidin (Schmelzpunkt: 214–216 °C) und 2-Hydroxyäthylamin.

Schmelzpunkt: 168–170 °C (Umfällung aus 2 n-Salzsäure mittels Ammoniak).

Beispiel 120
8-[N-(3,4-Dimethoxyphenäthyl)-methylamino]-2-(2-hydroxyäthylamino)-4-(1-oxido-thiomorpholino)-pyrimido[5,4-d]pyrimidin

Hergestellt analog Beispiel 2 aus 2-Chlor-8-[N-(3,4-dimethoxyphenäthyl)-methylamino]-4-(1-oxido-thiomorpholino)-pyrimido-[5,4-d]pyrimidin (Schmelzpunkt: 178–179 °C) und 2-Hydroxyäthylamin.

Schmelzpunkt: 153–154 °C (Essigsäureäthylester).

Beispiel 121
2-(2-Hydroxyäthylamino)-4-(1-oxido-thiomorpholino)-8-piperidino-pyrimido[5,4-d]pyrimidin

Hergestellt analog Beispiel 3 aus 2-Chlor-4-(1-oxido-thiomorpholino)-8-piperidino-pyrimido[5,4-d]pyrimidin (Schmelzpunkt: 203–204 °C) und 2-Hydroxyäthylamin.

Schmelzpunkt: 165–167 °C.

Beispiel 122
2-(3-Hydroxypropylamino)-4-(1-oxido-thiomorpholino)-8-piperidino-pyrimido[5,4-d]pyrimidin

Hergestellt analog Beispiel 3 aus 2-Chlor-4-(1-oxido-thiomorpholino)-8-piperidino-pyrimido[5,4-d]pyrimidin und 3-Hydroxypropylamin.

Schmelzpunkt: 142–144 °C (Essigsäureäthylester).

Beispiel 123
2-(2-Hydroxypropylamino)-4-(1-oxido-thiomorpholino)-8-piperidino-pyrimido[5,4-d]pyrimidin

Hergestellt analog Beispiel 3 aus 2-Chlor-4-(1-oxido-thiomorpholino)-8-piperidino-pyrimido[5,4-d]pyrimidin und 2-Hydroxypropylamin.

Schmelzpunkt: 175–177 °C (Essigsäureäthylester).

Beispiel 124
2-(2,2-Dimethyl-3-hydroxypropylamino)-4-(1-oxido-thiomorpholino)-8-piperidino-pyrimido[5,4-d]pyrimidin

Hergestellt analog Beispiel 3 aus 2-Chlor-4-(1-oxido-thiomorpholino)-8-piperidino-pyrimido[5,4-d]pyrimidin und 2,2-Dimethyl-3-hydroxypropylamin.

Schmelzpunkt: 192–194 °C (Äthanol).

Beispiel 125
2-(2,3-Dihydroxypropylamino)-4-(1-oxido-thiomorpholino)-8-piperidino-pyrimido[5,4-d]-pyrimidin

Hergestellt analog Beispiel 3 aus 2-Chlor-4-(1-oxido-thiomorpholino)-8-piperidino-pyrimido [5,4-d]pyrimidin und 2,3-Dihydroxypropylamin.

Schmelzpunkt: 189–190 °C (Äthanol).

Beispiel 126
2-[N-(2-Hydroxyäthyl)-2-methoxyäthylamino]-4-(1-oxido-thiomorpholino)-8-piperidino-pyrimido[5,4-d]pyrimidin

Hergestellt analog Beispiel 3 aus 2-Chlor-4-(1-oxido-thiomorpholino)-8-piperidino-pyrimido[5,4-d]pyrimidin und N-(2-Hydroxyäthyl)-2-methoxäthylamin.

Schmelzpunkt: 133–135 °C (Essigsäureäthylester).

Beispiel 127
2-(2-Hydroxyäthylamino)-4-(1-oxido-thiomorpholino)-8-pyrrolidino-pyrimido[5,4-d]pyrimidin

Hergestellt analog Beispiel 3 aus 2-Chlor-4-(1-oxido-thiomorpholino)-8-pyrrolidino-pyrimido[5,4-d]pyrimidin (Schmelzpunkt: 243–244 °C) und 2-Hydroxyäthylamin.

Schmelzpunkt: 200–203 °C.

Beispiel 128
8-Hexamethylenimino-2-(2-hydroxyäthylamino)-4-(1-oxido-thiomorpholino)-pyrimido [5,4-d] pyrimidin

Hergestellt analog Beispiel 3 aus 2-Chlor-8-hexamethylenimino-4-(1-oxido-thiomorpholino)-pyrimido[5,4-d]pyrimidin (Schmelzpunkt: 206–207 °C) und 2-Hydroxyäthylamin.

Schmelzpunkt: 190–192 °C (Essigsäureäthylester).

Beispiel 129
2-Diäthanolamino-8-heptamethylenimino-4-(1-oxido-thiomorpholino)-pyrimido[5,4-d]pyrimidin

Hergestellt analog Beispiel 3 aus 2-Chlor-8-heptamethylenimino-4-(1-oxido-thiomorpholino)-pyrimido[5,4-d]pyrimidin (Schmelzpunkt: 214–215 °C) und Diäthanolamin.

Schmelzpunkt: 186–188 °C (Äthanol).

Beispiel 130
2-Diäthanolamino-8-(4-methylpiperidino)-4-(1-oxido-thiomorpholino)-pyrimido[5,4-d]pyrimidin

Hergestellt analog Beispiel 3 aus 2-Chlor-8-(4-methylpiperidino)-4-(1-oxido-thiomorpholino)-pyrimido[5,4-d]pyrimidin (Schmelzpunkt: 195–196 °C) und Diäthanolamin.

Schmelzpunkt: 175–177 °C (Äthanol).

Beispiel 131
2-Diäthanolamino-8-(3,5-dimethylpiperidino)-4-(1-oxido-thiomorpholino)-pyrimido [5,4-d] pyrimidin

Hergestellt analog Beispiel 2 aus 2-Chlor-8-(3,5-dimethylpiperidino)-4-(1-oxido-thiomorpholino)-pyrimido[5,4-d]pyrimidin Schmelzpunkt: 186–187 °C) und Diäthanolamin.

Schmelzpunkt: 215–217 °C (Äthanol).

Beispiel 132

8-(2-Äthylpiperidino)-2-diäthanolamino-4-(1-oxido-thiomorpholino)-pyrimido [5,4-d] pyrimidin

Hergestellt analog Beispiel 3 aus 8-(2-Äthylpiperidino)-2-chlor-4-(1-oxido-thiomorpholino)-pyrimido [5,4-d] pyrimidin (Schmelzpunkt: 171–174 °C) und Diäthanolamin.

Schmelzpunkt: 140–142 °C (Essigsäureäthylester).

Beispiel 133

2-Diäthanolamino-4-(1-oxido-thiomorpholino)-8-(1,2,5,6-tetrahydropyridino)-pyrimido [5,4-d] pyrimidin

Hergestellt analog Beispiel 3 aus 2-Chlor-4-(1-oxido-thiomorpholino)-8- (1,2,5,6- tetrahydropyridino)-pyrimido[5,4-d]pyrimidin (Schmelzpunkt: 140–143 °C (Äthanol).

Beispiel 134

8-[Bis-(2-methoxyäthyl)-amino]-2-diäthanolamino-4-(1-oxido-thiomorpholino)-pyrimido [5,4-d]pyrimidin

Hergestellt analog Beispiel 3 aus 8-[Bis-(2-methoxyäthyl)-amino]-2-chlor-4-(1-oxido-thiomorpholino)-pyrimido[5,4-d]pyrimidin (Schmelzpunkt: 136–138 °C) und Diäthanolamin.

Schmelzpunkt: 120–122 °C (Essigsäureäthylester).

Beispiel 135

2-Diäthanolamino-8-methoxy-4-(1-oxido-thiomorpholino)-pyrimido[5,4-d]pyrimidin

Hergestellt analog Beispiel 4 aus 2-Chlor-8-methoxy-4-(1-oxido-thiomorpholino)-pyrimido [5,4-d] pyrimidin (Schmelzpunkt: 218–221 °C) und Diäthanolamin.

Schmelzpunkt: 198–200 °C (Dioxan).

Beispiel 136

2-Diäthanolamino-8-methoxy-4-morpholino-pyrimido[5,4-d]pyrimidin

Hergestellt analog Beispiel 4 aus 2-Chlor-8-methoxy-4-morpholino-pyrimido[5,4-d]pyrimidin (Schmelzpunkt: 160–162 °C) und Diäthanolamin.

Schmelzpunkt: 172–174 °C (Wasser).

Beispiel 137

2-Diäthanolamino-4-(1-oxido-thiomorpholino)-8-propoxy-pyrimido-[5,4-d]pyrimidin

Hergestellt analog Beispiel 4 aus 2-Chlor-4-(1-oxido-thiomorpholino)-8-propoxy-pyrimido[5,4-d]pyrimidin (Schmelzpunkt: 205–207 °C und Diäthanolamin.

Schmelzpunkt: 195–197 °C (Essigsäureäthylester).

Beispiel 138

2-Diäthanolamino-8-(3-methyl-n-butyl)-oxy-4-(1-oxido-thiomorpholino)-pyrimido[5,4-d]-pyrimidin

Hergestellt analog Beispiel 4 aus 2-Chlor-8-(3-methyl-n-butyl)-oxy-4-(1-oxido-thiomorpholino)-pyrimido[5,4-d]pyrimidin (Schmelzpunkt: 143–145 °C) und Diäthanolamin.

Schmelzpunkt: 153–155 °C (Essigsäureäthylester).

Beispiel 139

2-Diäthanolamino-8-oxtyloxy-4-(1-oxido-thiomorpholino)-pyrimido[5,4-d]pyrimidin

Hergestellt analog Beispiel 4 aus 2-Chlor-8-octyloxy-4-(1-oxido-thiomorpholino)-pyrimido[5,4-d]pyrimidin (Schmelzpunkt: 94–96 °C) und Diäthanolamin.

Schmelzpunkt: 128–130 °C (Essigsäureäthylester).

Beispiel 140

clohexyloxy-2-diäthanolamino-4-(1-oxido-thiomorpholino)-pyrimido[5,4-d]pyrimidin

Hergestellt analog Beispiel 4 aus 2-Chlor-8-cyclohexyloxy-4-(1-oxido-thiomorpholino)-pyrimido [5,4-d] pyrimidin(Schmelzpunkt: 185–187 °C) und Diäthanolamin.

Schmelzpunkt: 217–219 °C (Äthanol).

Beispiel 141

8-Benzyloxy-2-(2-hydroxyäthylamino)-4-(1-oxido-thiomprpholino)-pyrimido [5,4-d] pyrimidin

Hergestellt analog Beispiel 4 aus 8-Benzyloxy-2-chlor-4-(1-oxido-thiomorpholino)-pyrimido [5,4-d] pyrimidin (Schmelzpunkt: 227–229 °C) und 2-Hydroxyäthylamin.

Schmelzpunkt: 182–184 °C (Essigsäureäthylester/Methanol)

Beispiel 142

8-Benzyloxy-2-(3-hydroxypropylamino)-4-(1-xido-thiomorpholino)-pyrimido [5,4-d] pyrimidin

Hergestellt analog Beispiel 4 aus 8-Benzyloxy-2-chlor-4-(1-oxido-thiomorpholino)-pyrimido [5,4-d] pyrimidin und 3-Hydroxypropylamin.

Schmelzpunkt: 179–181 °C (Äthanol).

Beispiel 143

8-Benzyloxy-2-[N-(2-hydroxyäthyl)-5-hydroxypentylamino]-4-(1-oxido-thiomorpholino)-pyrimido[5,4-d]pyrimidin

Hergestellt analog Beispiel 4 aus 8-Benzyloxy-2-chlor-4-(1-oxido-thiomorpholino)-pyrimido [5,4-d]pyrimidin und N-(2-Hydroxyäthyl)-5-hydroxypentylamin.

Schmelzpunkt: 120–122 °C (Essigsäureäthylester).

Beispiel 144
8-Benzyloxy-2-(N-methylglucamino)-4-(1-oxido-thiomorpholino)-pyrimido[5,4-d]pyrimidin

Hergestellt analog Beispiel 4 aus 8-Benzyloxy-2-chlor-4-(1-oxido-thiomorpholino)-pyrimido[5,4-d]pyrimidin und N-Methylglucamin.

Schmelzpunkt: 199–201 °C (Äthanol).

Beispiel 145
8-Benzyloxy-2-[N-(2-hydroxyäthyl)-2-methoxyäthylamino]-4-(1-oxido-thiomorpholino)-pyrimido[5,4-d]pyrimidin

Hergestellt analog Beispiel 4 aus 8-Benzyloxy-2-chlor-4-(1-oxido-thiomorpholino)-pyrimido[5,4-d]pyrimidin und N-(2-Hydroxyäthyl)-2-methoxyäthylamin.

Schmelzpunkt: 110–112 °C (Methanol/Wasser).

Beispiel 146
8-Benzyloxy-2-[N-(2-hydroxyäthyl)-2-hydroxypropylamino]-4-(1-oxido-thiomorpholino) pyrimido[5,4-d]pyrimidin

Hergestellt analog Beispiel 4 aus 8-Benzyloxy-2-chlor-4-(1-oxido-thiomorpholino)-pyrimido[5,4-d]pyrimidin und N-(2-Hydroxyäthyl)-2-hydroxypropylamin.

Schmelzpunkt: 148–150 °C (Essigsäureäthylester).

Beispiel 147
8-Benzyloxy-2-diisopropanolamino-4-(1-oxido-thiomorpholino)-pyrimido[5,4]pyrimidin

Hergestellt analog Beispiel 4 aus 8-Benzyloxy-2-chlor-4-(1-oxido-thiomorpholino)-pyrimido[5,4-d]pyrimidin und Diisopropanolamin (= Bis-(2-hydroxypropyl)-amin

Schmelzpunkt: 199–201 °C (Essigsäureäthylester).

Beispiel 148
8-Benzyloxy-2-[N-(2-hydroxyäthyl)-2,3-dihydroxypropylamino]-4-(1-oxido-thiomorpholino)-pyrimido[5,4-d]pyrimidin

Hergestellt analog Beispiel 4 aus 8-Benzyloxy-2-chlor-4-(1-oxido-thiomorpholino)-pyrimido[5,4-d]pyrimidin und N-(2-Hydroxyäthyl)-2,3-dihydroxypropylamin.

Schmelzpunkt: 163–165 °C (Essigsäureäthylester).

Beispiel 149
8-Benzyloxy-2-(3-hydroxypiperidino)-4-(1-oxido-thiomorpholino)-pyrimido[5,4-d] pyrimidin

Hergestellt analog Beispiel 4 aus 8-Benzyloxy-2-chlor-4-(1-oxido-thiomorpholino)-pyrimido[5,4-d]pyrimidin und 3-Hydroxypiperidin.

Schmelzpunkt: 210–212 °C (Äthanol).

Beispiel 150
2-(3-Hydroxypropylamino)-4-(1-oxido-thiomorpholino)-8-phenäthoxy-pyrimido[5,4-d] pyrimidin

Hergestellt analog Beispiel 4 aus 2-Chlor-4-(1-oxido-thiomorpholino)-8-phenäthoxy-pyrimido[5,4-d]pyrimidin (Schmelzpunkt: 242–245 °C) und 3-Hydroxypropylamin in Dimethylsulfoxid.

Schmelzpunkt: 174–176 °C (Methanol).

Beispiel 151
2-Diäthanolamino-4-(1-oxido-thiomorpholino)-8-phenäthoxy-pyrimido[5,4-d]pyrimidin

Hergestellt analog Beispiel 4 aus 2-Chlor-4-(1-oxido-thiomorpholino)-8-phenäthoxy-pyrimido [5,4-d]pyrimidin und Diäthanolamin.

Schmelzpunkt: 152–154 °C (Methanol).

Beispiel 152
2-(3-Hydroxypiperidino)-4-(1-oxido-thiomorpholino)-8-phenäthoxy-pyrimido[5,4-d] pyrimidin

Hergestellt analog Beispiel 4 aus 2-Chlor-4-(1-oxido-thiomorpholino)-8-phenäthoxy-pyrimido[5,4-d]pyrimidin und 3-Hydroxypiperidin.

Schmelzpunkt: 192–194 °C (Methanol).

Beispiel 153
2-Diäthanolamino-4-(1-oxido-thiomorpholino)-8-(3-phenylpropoxy)-pyrimido[5,4-d] pyrimidin

Hergestellt analog Beispiel 4 aus 2-Chlor-4-(1-oxido-thiomorpholino)-8-(3-phenylpropoxy)-pyrimido[5,4-d]pyrimidin (Schmelzpunkt: 152–154 °C) und Diäthanolamin.

Schmelzpunkt: 159–161 °C (Essigsäureäthylester).

Beispiel 154
2-Diäthanolamino-4-(1-oxido-thiomorpholino)-8-(4-phenylbutoxy-pyrimido[5,4-d] pyrimidin

Hergestellt analog Beispiel 4 aus 2-Chlor-4-(1-oxido-thiomorpholino)-8-(4-phenylbutoxy)-pyrimido[5,4-d]pyrimidin (Schmelzpunkt: 124–126 °C) und Diäthanolamin.

Schmelzpunkt: 98–100 °C (Essigsäureäthylester).

Beispiel 155
8-Benzyloxy-2-diäthanolamino-4-morpholino-pyrimido[5,4-d]pyrimidin

Hergestellt analog Beispiel 4 aus 8-Benzyloxy-2-chlor-4-morpholino-pyrimido[5,4-d]pyrimidin (Schmelzpunkt: 146–148 °C) und Diäthanolamin.

Schmelzpunkt: 167–169 °C (Essigsäureäthylester).

Beispiel 156
8-Benzyloxy-2-diäthanolamino-4-thiomorpholino-pyrimido[5,4-d]pyrimidin

Hergestellt analog Beispiel 4 aus 8-Benzyloxy-2-chlor-4-thiomorpholino-pyrimido[5,4-d] pyrimidin (Schmelzpunkt: 183–185 °C) und Diäthanolamin.

Schmelzpunkt: 120–122 °C (Essigsäureäthylester).

Beispiel 157

8-Benzyloxy-2-dimethylamino-4- (1-oxido-thiomorpholino)- pyrimido[5,4-d]pyrimidin

Hergestellt analog Beispiel 4 aus 8-Benzyloxy-2-chlor-4- (1-oxido-thiomorpholino)- pyrimido[5,4-d]pyrimidin (Schmelzpunkt: 227–229 °C) und Dimethylamin unter Druck.

Schmelzpunkt: 202–204 °C (Äthanol).

Beispiel 158

2-Diäthanolamino-8-furfuryloxy-4- (1-oxido-thiomorpholino)- pyrimido[5,4-d]pyrimidin

Hergestellt analog Beispiel 4 aus 2-Chlor-8-furfuryloxy-4- (1-oxido-thiomorpholino)- pyrimido[5,4-d]pyrimidin (Schmelzpunkt: 183–185 °C) und Diäthanolamin.

Schmelzpunkt: 198–200 °C (Dioxan).

Beispiel 159

8-(3-Äthoxypropoxy)-2- diäthanolamino-4- (1-oxido-thiomorpholino)- pyrimido[5,4-d]pyrimidin

Hergestellt analog Beispiel 4 aus 8-(3-Äthoxypropoxy) -2-chlor-4- (1-oxido-thiomorpholino)pyrimido[5,4-d]pyrimidin (Schmelzpunkt: 130–133 °C) und Diäthanolamin.

Schmelzpunkt: 131–133 °C (Essigsäureäthylester).

Beispiel 160

2-Diäthanolamino -8- (4-methylbenzyloxy) -4-(1-oxido-thiomorpholino)- pyrimido[5,4-d]pyrimidin

Hergestellt analog Beispiel 4 aus 2-Chlor-8-(4-methylbenzyloxy)- 4- (1-oxido-thiomorpholino)- pyrimido[5,4-d]pyrimidin (Schmelzpunkt: 178–180 °C) und Diäthanolamin.

Schmelzpunkt: 171–173 °C (Methanol.

Beispiel 161

2-Diäthanolamino-8- (4-fluorbenzyloxy) -4- (1-oxido-thiomorpholino)- pyrimido[5,4-d]pyrimidin

Hergestellt analog Beispiel 4 aus 2-Chlor-8-(4-fluorbenzyloxy)-4- (1-oxido-thiomorpholino)- pyrimido[5,4-d]pyrimidin (Schmelzpunkt: 226–228 °C) und Diäthanolamin.

Schmelzpunkt: 186–188 °C (Äthanol).

Beispiel 162

8-(4-Chlorbenzyloxy)- 2-diäthanolamino-4- (1-oxido-thiomorpholino)- pyrimido[5,4-d]pyrimidin

Hergestellt analog Beispiel 4 aus 2-Chlor-8-(4-chlorbenzyloxy)-4- (1-oxido-thiomorpholino)pyrimido[5,4-d]pyrimidin (Schmelzpunkt: 214–216 °C) und Diäthanolamin.

Schmelzpunkt: 198–200 °C (Essigsäureäthylester/Methanol).

Beispiel 163

2-Diäthanolamino-8- (2,4-dichlorbenzyloxy) -4-(1-oxido-thiomorpholino)-pyrimido[5,4-d]pyrimidin

Hergestellt analog Beispiel 4 aus 2-Chlor-8-(2,4-dichlorbenzyloxy)-4- (1-oxido-thiomorpholino)- pyrimido[5,4-d] pyrimidin (Schmelzpunkt: 249–251 °C) und Diäthanolamin.

Schmelzpunkt: 208–210 °C (Äthanol/Essigsäureäthylester).

Beispiel 164

2-Diäthanolamin-8- (2-methoxybenzyloxy) -4-(1-oxido-thiomorpholino)- pyrimido[5,4-d]pyrimidin

Hergestellt analog Beispiel 4 aus 2-Chlor-8-(2-methoxybenzyloxy) -4-(1-oxido-thiomorpholino)- pyrimido[5,4-d]pyrimidin (Schmelzpunkt: 223–225 °C) und Diäthanolamin.

Schmelzpunkt: 174–176 °C (Essigsäureäthylester/Methanol).

Beispiel 165

2-Diäthanolamin-8- (4-methoxybenzyloxy) -4-(1-oxido-thiomorpholino)- pyrimido[5,4-d]pyrimidin

Hergestellt analog Beispiel 4 aus 2-Chlor-8-(4-methoxybenzyloxy) -4- (1-oxido-thiomorpholino)- pyrimido[5,4-d]pyrimidin (Schmelzpunkt: 184–186 °C) und Diäthanolamin.

Schmelzpunkt: 147–149 °C (Essigsäureäthylester/Methanol)

Beispiel 166

2-Diäthanolamino-8-(3,4-dimethoxybenzyloxy)-4-(1-oxido-thiomorpholino)-pyrimido[5,4-d]pyrimidin

Hergestellt analog Beispiel 4 aus 2-Chlor-8-(3,4-dimethoxybenzyloxy) -4- (1-oxido-thiomorpholino)- pyrimido[5,4-d]pyrimidin (Schmelzpunkt: 171–173 °C) und Diäthanolamin.

Schmelzpunkt: 157–159 °C (Dioxan).

Beispiel 167

2-Diäthanolamino-8- (3,4-methylendioxy-benzyloxy) -4- (1-oxido-thiomorpholino) -pyrimido[5,4-d]pyrimidin

Hergestellt analog Beispiel 4 aus 2-Chlor-8-(3,4-methylendioxybenzyloxy) -4- (1-oxido-thiomorpholino)- pyrimido[5,4-d]pyrimidin (Schmelzpunkt: 216–218 °C) und Diäthanolamin.

Schmelzpunkt: 186–187 °C (Dioxan).

Beispiel 168

2-(2-Hydroxyäthylamino) -8- (4-methylbenzylthio) -4- (1-oxido-thiomorpholino)- pyrimido[5,4-d]pyrimidin

Hergestellt analog Beispiel 1 aus 2-Chlor-8-(4-methylbenzylthio) -4- (1-oxido-thiomorpholino)- pyrimido[5,4-d]pyrimidin (Schmelzpunkt: 262–264 °C) und 2-Hydroxyäthylamin.

Schmelzpunkt: 189–191 °C (Dioxan).

Beispiel 169

8-(4-Fluorbenzylthio) -2- (2-hydroxyäthylamino) -4- (1-oxido-thiomorpholino)- pyrimido[5,4-d]pyrimidin

Hergestellt analog Beispiel 1 aus 2-Chlor-8-(4-fluorbenzylthio) -4- (1-oxido-thiomorpholino)-pyrimido[5,4-d]pyrimidin (Schmelzpunkt: 192–194 °C) und 2-Hydroxyäthylamin.
Schmelzpunkt: 222–224 °C (Äthanol/Dioxan).

Beispiel 170
8-(2-Chlorbenzylthio)-2-(2-hydroxyäthylamino)-4(1-oxido-thiomorpholino)-pyrimido[5,4-d]pyrimidin
Hergestellt analog Beispiel 1 aus 2-Chlor-8-(2-chlorbenzylthio)-4-(1-oxido-thiomorpholino)-pyrimido[5,4-d]pyrimidin (Schmelzpunkt: 203–206 °C) und 2-Hydroxyäthylamin.
Schmelzpunkt: 205–207 °C (Äthanol/Dioxan).

Beispiel 171
8-(4-Chlorbenzylthio)-2-(2-hydroxyäthylamino)-4-(1-oxido-thiomorpholino)-pyrimido[5,4-d]pyrimidin
Hergestellt analog Beispiel 1 aus 2-Chlor-8-(4-chlorbenzylthio)-4-(1-oxido-thiomorpholino)-pyrimido[5,4-d]pyrimidin (Schmelzpunkt: 232–234 °C) und 2-Hydroxyäthylamin.
Schmelzpunkt: 222–224 °C (Äthanol/Dioxan).

Beispiel 172
8-(2,4-Dichlorbenzylthio)-2-(2-hydroxyäthylamino)-4-(1-oxido-thiomorpholino)-pyrimido[5,4-d]pyrimidin
Hergestellt analog Beispiel 1 aus 2-Chlor-8-(2,4-dichlorbenzylthio)-4-(1-oxido-thiomorpholino)-pyrimido[5,4-d]pyrimidin (Schmelzpunkt: 227–229 °C) und 2-Hydroxyäthylamin.
Schmelzpunkt: 254–256 °C (Dioxan).

Beispiel 173
8-(3,4-Dichlorbenzylthio)-2-(2-hydroxyäthylamino)-4-(1-oxido-thiormopholino)-pyrimido[5,4-d]pyrimidin
Hergestellt analog Beispiel 1 aus 2-Chlor-8-(3,4-dichlorbenzylthio)-4-(1-oxido-thiomorpholino)-pyrimido[5,4-d]pyrimidin (Schmelzpunkt: 210–212 °C) und 2-Hydroxyäthylamin.
Schmelzpunkt: 211–213 °C (Äthanol/Dioxan).

Beispiel 174
2-(2-Hydroxyäthylamino)-4-(1-oxido-thiomorpholino)-8-(3-trifluormethyl-benzylthio)-pyrimido[5,4-d]pyrimidin
Hergestellt analog Beispiel 1 aus 2-Chlor-4-(1-oxido-thiomorpholino)-8-(trifluormethylbenzylthio)-pyrimido[5,4-d]pyrimidin (Schmelzpunkt: 211–213 °C) und 2-Hydroxyäthylamin.
Schmelzpunkt: 199–201 °C (Essigsäureäthylester).

Beispiel 175
2-(2-Hydroxyäthylamino)-8-(4-methoxybenzylthio)-4-(1-oxido-thiomorpholino)-pyrimido[5,4-d]pyrimidin
Hergestellt analog Beispiel 1 aus 2-Chlor-8-(4-methoxybenzylthio)-4-(1-oxido-thiomorpholino)-pyrimido[5,4-d]pyrimidin (Schmelzpunkt: 217–219 °C) und 2-Hydroxyäthylamin.
Schmelzpunkt: 204–206 °C (Äthanol/Dioxan).

Beispiel 176
8-(3,4-Dimethoxybenzylthio)-2-(2-hydroxyäthylamino)-4-(1-oxido-thiomorpholino)-pyrimido[5,4-d]pyrimidin
Hergestellt analog Beispiel 1 aus 2-Chlor-8-(3,4-dimethoxybenzylthio)-4-(1-oxido-thiomorpholino)-pyrimido[5,4-d]pyrimidin (Schmelzpunkt: 186–188 °C) und 2-Hydroxyäthylamin.
Schmelzpunkt: 198–200 °C (Äthanol).

Beispiel 177
2-(2-Hydroxyäthylamino)-8-(3,4-methylendioxy-benzylthio)-4-(1-oxido-thiomorpholino)-pyrimido[5,4-d]pyrimidin
Hergestellt analog Beispiel 1 aus 2-Chlor-8-(3,4-methylendioxybenzylthio)-4-(1-oxido-thiomorpholino)-pyrimido[5,4-d]pyrimidin (Schmelzpunkt: 229–231 °C) und 2-Hydroxyäthylamin.
Schmelzpunkt: 215–217 °C (Äthanol/Dioxan).

Beispiel 178
2-(2-Hydroxyäthylamino)-8-(α-methyl-4-methylthio-benzylthio)-4-(1-oxido-thiomorpholino)-pyrimido[5,4-d]pyrimidin
Hergestellt analog Beispiel 1 aus 2-Chlor-8-(α-methyl-4-methylthio-benzylthio)-4-(1-oxido-thiomorpholino)-pyrimido[5,4-d]pyrimidin (Schmelzpunkt: 200–202 °C) und 2-Hydroxyäthylamin.
Schmelzpunkt: 150–152 °C (Äthanol).

Beispiel 179
2-(2-Hydroxyäthylamino)-8-(α-methyl-4-methylthio-benzylthio)-4-morpholino-pyrimido[5,4-d] pyrimidin
Hergestellt analog Beispiel 1 aus 2-Chlor-8-(α-methyl-4-methylthio-benzylthio)-pyrimido[5,4-d] pyrimidin und 2-Hydroxyäthylamin.
Schmelzpunkt: 142–144 °C (Äthanol)

Beispiel 180
8-(3-Chlorbenzylthio)-2-(2-hydroxyäthylamino)-4-(1-oxido-thimorpholino)-pyrimido-[5,4-d]pyrimidin
Hergestellt analog Beispiel 1 aus 2-Chlor-8-(3-chlorbenzylthio)-4-(1-oxido-thiomorpholino)-pyrimido[5,4-d]pyrimdin (Schmelzpunkt: 179–182 °C) und 2-Hydroxyäthylamin.
Schmelzpunkt: 200–202 °C (Äthanol/Dioxan)

Beispiel 181
8-(2,6-Dichlorbenzylthio)-2-(2-hydroxyäthylamino)-4-(1-oxido-thiomorpholino)-pyrimido[5,4-d]pyrimidin
Hergestellt analog Beispiel 1 aus 2-Chlor-8-(2,6-dichlorbenzylthio)-4-(1-oxido-thiomorpholino)-pyrimido[5,4-d]pyrimidin (Schmelzpunkt: 215–218 °C) und 2-Hydroxyäthylamin.
Schmelzpunkt: 203–206 °C (Äthanol/Dioxan)

Beispiel 182
2-[N-Äthyl-(2-hydroxyäthylamino)]-8-benzylthio-4-(1-oxido-thiomorpholino)-pyrimido-[5,4-d]pyrimidin

Hergestellt analog Beispiel 1 aus 8-Benzylthio-2-chlor-4-(1-oxido-thiomorpholino)pyrimido[5,4-d]pyrimidin (Schmelzpunkt: 188–190 °C) und N-Äthyl-2-hydroxyäthylamin.

Schmelzpunkt: 182–184 °C (Methanol).

Beispiel 183

8-Benzylthio-2-[N-(2-hydroxyäthyl)-isopropylamino]-4-(1-oxido-thiomorpholino-pyrimido[5,4-d]pyrimidin

Hergestellt analog Beispiel 1 aus 8-Benzylthio-2-chlor-4-(1-oxido-thiomorpholino)-pyrimido[5,4-d]pyrimidin und N-(2-Hydroxyäthyl)-isopropylamin.

Schmelzpunkt: 188–190 °C (Essigsäureäthylester)

Beispiel 184

8-Benzylthio-2-[N-butylamino-(2-hydroxyäthylamino)-4-(1-oxido-thiomorpholino)-pyrimido[5,4-d]pyrimidin

Hergestellt analog Beispiel 1 aus 2-Benzylthio-2-chlor-4-(1-oxido-thiomorpholino)-pyrimido[5,4-d]pyrimidin und N-Butyl-2-hydroxyäthylamin.

Schmelzpunkt: 111–113 °C (Essigsäureäthylester)

Beispiel 185

8-Benzylthio-2-(1,1-dimethyl-2-hydroxyäthylamino)-4-(1-oxido-thiomorpholino)-pyrimido [5,4-d] pyrimidin

Hergestellt analog Beispiel 1 aus 8-Benzylthio-2-chlor-4-(1-oxido-thiomorpholino)-pyrimido[5,4-d]pyrimidin (Schmelzpunkt: 188–190 °C) und 2-Amino-2-methyl-propanol in Dioxan durch 24-stündiges Erhitzen unter Rückfluss.

Schmelzpunkt: 186–188 °C (Äthanol).

Beispiel 186

8-Benzylthio-2-(2,2-dimethyl-3-hydroxypropylamino)-4-(1-oxido-thiomorpholino)-pyrimido [5,4-d]pyrimidin

Hergestellt analog Beispiel 1 aus 8-Benzylthio-2-chlor-4-(1-oxido-thiomorpholino)-pyrimido [5,4-d]pyrimidin und 3-Amino-2,2-dimethylpropanol.

Schmelzpunkt: 181–183 °C (Äthanol)

Beispiel 187

8-(2-Äthyl-hexylamino)-2-(2-hydroxyäthylamino)-4-(1-oxido-thiomorpholino)-pyrimido[5,4-d] pyrimidin

Hergestellt analog Beispiel 2 aus 8-(2-Äthylhexylamino)-2-chlor-4-(1-oxido-thiomorpholino)-pyrimido[5,4-d]pyrimidin (Schmelzpunkt: 118–120 °C) und 2-Hydroxyäthylamin.

Schmelzpunkt: 140–142 °C (Essigsäureäthylester)

Beispiel 188

8-(2-Chloranilino)-2-(2-hydroxyäthylamino)-4-(1-oxido-thiomorpholino)-pyrimido[5,4-d]pyrimidin

Hergestellt analog Beispiel 3 aus 2-Chlor-8-(2-chloranilino)-4-(1-oxido-thiomorpholino)-pyrimido[5,4-d]pyrimidin (Schmelzpunkt: 263–265 °C) und 2-Hydroxyäthylamin.

Schmelzpunkt: 216–218 °C (Dioxan)

Beispiel 189

8-(2,6-Dichlorbenzylamino)-2-(2-hydroxyäthylamino)-4-(1-oxido-thiomorpholino)-pyrimido[5,4-d]pyrimidin

Hergestellt analog Beispiel 2 aus 2-Chlor-8-(2,6-dichlorbenzylamino)-4-(1-oxido-thiomorpholino)-pyrimido[5,4-d]pyrimidin (Schmelzpunkt: 239–241 °C) und 2-Hydroxyäthylamin.

Schmelzpunkt: 199–201 °C (Äthanol)

Beispiel 190

8-Benzylamino-2-[N-(2-hydroxyäthyl)-methylamino]-4-(1-oxido-thiomorpholino)-pyrimido[5,4-d]pyrimidin

Hergestellt analog Beispiel 2 aus 8-Benzylamino-2-chlor-4-(1-oxido-thiomorpholino)-pyrimido [5,4-d]pyrimidin (Schmelzpunkt: 232–233 °C) und N-(2-Hydroxyäthyl)-methylamin in Dioxan unter Rückfluss.

Schmelzpunkt: 171–173 °C (Menthol)

Beispiel 191

2-[N-Äthyl-(2-hydroxyäthylamino)]-8-benzylamino-4-(1-oxido-thiomorpholino)-pyrimido [5,4-d]pyrimidin

Hergestellt analog Beispiel 2 aus 8-Benzylamino-2-chlor-4-(1-oxido-thiomorpholino)-pyrimido[5,4-d]pyrimidin und N-Äthyl-2-hydroxyäthylamin.

Schmelzpunkt: 175–177 °C (Essigsäureäthylester)

Beispiel 192

8-Benzylamino-2-[N-(2-hydroxyäthyl)-propylamino]-4-(1-oxido-thiomorpholino)-pyrimido [5,4-d]pyrimidin

Hergestellt analog Beispiel 2 aus 8-Benzylamino-2-chlor-4-(1-oxido-thiomorpholino)-pyrimido [5,4-d]pyrimidin und N-(2-Hydroxyäthyl)-propylamin.

Schmelzpunkt: 143–145 °C (Essigsäureäthylester)

Beispiel 193

8-Benzylamino-2-cyclohexylamino-4-(1-oxido-thiomorpholino)-pyrimido[5,4-d]pyrimidin

Hergestellt analog Beispiel 2 aus 8-Benzylamino-2-chlor-4-(1-oxido-thiomorpholino)-pyrimido [5,4-d]pyrimidin und Cyclohexyl-amin.

Schmelzpunkt: 174–176 °C (Methanol)

Beispiel 194

2-Diäthanolamino-8-(2-fluorbenzyloxy)-4-(1-

oxido-thiomorpholino)-pyrimido[5,4-d]pyrimidin

Hergestellt analog Beispiel 4 aus 2-Chlor-8-(2-fluorbenzyloxy)-4-(1-oxido-thiomorpholino)-pyrimido[5,4-d]pyrimidin (Schmelzpunkt: 212–214 °C) und Diäthanolamin.

Schmelzpunkt: 150–152 °C (Essigsäureäthylester)

Beispiel 195
2-Diäthanolamino-8-(3-fluorbenzyloxy)-4-(1-oxido-thiomorpholino)-pyrimido[5,4-d]pyrimidin

Hergestellt analog Beispiel 4 aus 2-Chlor-8-(3-fluorbenzyloxy)-4-(1-oxido-thiomorpholino)-pyrimido[5,4-d]pyrimidin (Schmelzpunkt: 206–208 °C) und Diäthanolamin.

Schmelzpunkt: 190–192 °C (Dioxan)

Beispiel 196
2-Diäthanolamino-8-(2-chlorbenzyloxy)-4-(1-oxido-thiomorpholino)-pyrimido[5,4-d]pyrimidin

Hergestellt analog Beispiel 4 aus 2-Chlor-8-(2-chlorbenzyloxy)-4-(-oxido-thiomorpholino)-pyrimido[5,4–d]pyrimidin (Schmelzpunkt: 213–215 °C) und Diäthanolamin.

Schmelzpunkt: 209–211 °C (Essigsäureäthylester/Methanol).

Beispiel 197
2-Diäthanolamino-8-(2,6-dichlorbenzyloxy)-4-(1-oxido-thiomorpholino)-pyrimido[5,4-d]pyrimidin

Hergestellt analog Beispiel 4 aus 2-Chlor-8-(2,6-dichlorbenzyloxy)-4-(1-oxido-thiomorpholino)-pyrimido[5,4-d]pyrimidin (Schmelzpunkt: 237–239 °C) und Diäthanolamin.

Schmelzpunkt: 206–208 °C (Methanol/Essigsäureäthylester)

Beispiel 198
2-Diäthanolamino-8-(3,4-dichlorbenzyloxy)-4-(1-oxido-thiomorpholino)-pyrimido[5,4-d]pyrimidin

Hergestellt analog Beispiel 4 aus 2-Chlor-8-(3,4-dichlorbenzyloxy)-4-(1-oxido-thiormorpholino)-pyrimido[5,4-d]pyrimidin (Schmelzpunkt: 211–213 °C) und Diäthanolamin.

Schmelzpunkt: 205–207 °C (Dioxan)

Beispiel 199
2-Diäthanolamino-8-(2,2-dimethylpropoxy)-4-(1-oxido-thiomorpholino)-pyrimido[5,4-d]pyrimidin

Hergestellt analog Beispiel 4 aus 2-Chlor-8-(2,2-dimethylpropoxy)-4-(1-oxido-thiomorpholino)-pyrimido[5,4-d]pyrimidin (Schmelzpunkt: 231–232 °C) und Diäthanolamin.

Schmelzpunkt: 171–173 °C (Essigsäureäthylester)

Beispiel 200
8-Benzyloxy-2-(N-methyl-3-picolylamino)-4-(1-oxido-thiomorpholino)-pyrimido[5,4-d]pyrimidin

Hergestellt analog Beispiel 4 aus 8-Benzyloxy-2-chlor-4-(1-oxido-thiomorpholino)-pyrimido[5,4-d]pyrimidin (Schmelzpunkt: 227–229 °C) und N-Methyl-3-picolylamin.

Schmelzpunkt: 168–170 °C (Essigsäureäthylester/Methanol)

Beispiel A
Dragées mit 1 mg 8-Benzylthio-2-(2-hydroxyäthylamino)-4-(1-oxido-thiomorpholino)-pyrimido[5,4-d]pyrimidin

Zusammensetzung:
1 Dragéekern enthält

| | | |
|---|---|---|
| Wirksubstanz | (1) | 1,0 mg |
| Milchzucker | (2) | 30,0 mg |
| Maisstärke | (3) | 14,5 mg |
| Polyvinylpyrrolidon | (4) | 4.0 mg |
| Magnesiumstearat | (5) | 0,5 mg |
| | | 50,0 mg |

Herstellung:
Die Stoffe (1)–(3) werden mit einer wässrigen Lösung von (4) gleichmässig befeuchtet, durch 1 mm-Maschenweite gesiebt, getrocknet und erneut durch 1 mm-Maschenweite gesiebt. Nach Zumischen von (5) wird die Mischung zu Dragéekernen verpresst.

Dragéekerne: 5 mm ∅, bikonvex, rund

Dragierung:
Übliche Zuckerdragierung auf 70 mg Endgewicht.

Beispiel B
Tabletten mit 2 mg 8-Benzylthio-2-(2-hydroxyäthylamino)-4-(1-oxido-thiomorpholino)-pyrimido[5,4-d]pyrimidin

1 Tablette enthält:

| | |
|---|---|
| Wirksubstanz | 2,0 mg |
| Milchzucker | 29,0 mg |
| Maisstärke | 14,5 mg |
| Polyvinylpyrrolidon | 4,0 mg |
| Magnesiumstearat | 0,5 mg |
| | 50,0 mg |

Herstellung:
Analog den Dragéekernen.

Tablettenbeschreibung:

Gewicht: 50 mg
Durchmesser: 5 mm, biplan, beidseitige Facette

Beispiel C
Suppositorien zu 5 mg 8-Benzylthio-2-(2-hydroxyäthylamino)-4-(1-oxido-thiomorpholino)-pyrimido[5,4-d]pyrimidin

1 Zäpfchen enthält:

| | |
|---|---|
| Wirksubstanz | 0,005 g |
| Hartfett (z.B. Witepsol H 19 und Witepsol W 45) | 1,695 g |
| | 1,700 g |

Herstellung:
Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.
Zäpfchengewicht: 1,7 g

Beispiel D
Suspension mit 2 mg 8-Benzylthio-2-(2-hydroxyäthylamino)-4-(1-oxido-thiomorpholino)-pyrimido[5,4-d]pyrimidin pro 5 ml

100 ml Suspension enthalten:

| | |
|---|---|
| Wirksubstanz | 0,04 g |
| Carboxymethylcellulose | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70% | 20,0 g |
| Aroma | 0,3 g |
| Wasser dest. ad | 100,0 ml |

Herstellungsverfahren:
Dest. Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

Beispiel E
Ampullen mit 1 mg 2-Diäthanolamino-4-(1-oxido-thiomorpholino)-8-piperidino-pyrimido[5,4-d]pyrimidin

1 Ampulle enthält:

| | |
|---|---|
| Wirksubstanz | 1,0 mg |
| Salzsäure 0,01 N | 0,3 ml |
| NaCl | 18,0 mg |
| Wasser für Injektionszwecke ad | 2,0 ml |

Herstellung:
In einem geeichten Ansatzgefäss wird die Wirkstoffbase in Wasser für Injektionszwecke suspendiert und unter Erwärmen und Zutropfen von Salzsäure vollständig gelöst. Nach Filtration über Membranfilter wird die Lösung in Ampullen abgefüllt und im Autoklaven sterilisiert.

**Patentansprüche**

1. Trisubstituierte Pyrimido[5,4-d]-pyrimidine der allgemeinen Formel (I)

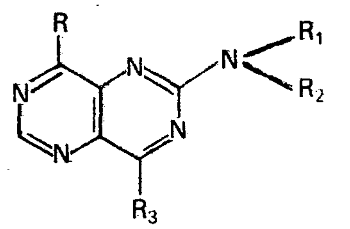

(I)

in der
$R_1$ eine gegebenenfalls ab Kohlenstoffatom 2 mit bis zu 5 Hydroxygruppen substituierte geradkettige oder verzweigte Alkylgruppe mit 2 bis 8 Kohlenstoffatomen, eine durch eine Alkoxy-, Amino-, Alkylamino-, Dialkylamino- oder Morpholinogruppe in 2-, 3- oder 4-Stellung substituierte Alkylgruppe mit 2 bis 4 Kohlenstoffatomen, wobei die vorstehend erwähnten Alkylsubstituenten jeweils 1 bis 3 Kohlenstoffatome enthalten können, eine Methyl- oder Pyridylmethylgruppe,
$R_2$ ein Wasserstoffatom, eine Alkylgruppe mit 1 bis 3 Kohlenstoffatomen oder eine durch eine Hydroxygruppe oder eine Alkoxygruppe mit 1 bis 3 Kohlenstoffatomen in 2- oder 3-Stellung substituierte Alkylgruppe mit 2 oder 3 Kohlenstoffatomen oder
$R_1$ und $R_2$ zusammen mit dem dazwischenliegenden Stickstoffatom eine Imidazolyl-, Morpholino-, Thiomorpholino-, 1-Oxidothiomorpholino- oder Piperidinogruppe, wobei die Piperidinogruppe durch eine Hydroxygruppe, eine Alkoxygruppe mit 1 bis 3 Kohlenstoffatomen, eine Hydroxyalkylgruppe mit 1 bis 3 Kohlenstoffatomen, eine Alkoxycarbonylgruppe mit insgesamt 2 bis 4 Kohlenstoffatomen, eine Aminogruppe, eine Alkanoylaminogruppe mit 1 bis 3 Kohlenstoffatomen im Alkanoylteil, eine Aminocarbonyl-, Alkylamino- oder Dialkylaminogruppe mit jeweils 1 bis 3 Kohlenstoffatomen im Alkylteil substituiert sein kann,
$R_3$ eine gegebenenfalls durch eine oder zwei Alkylgruppen mit jeweils 1 bis 3 Kohlenstoffatomen substituierte Morpholino-, Thiomorpholino-, 1-Oxido-thiomorpholino- oder 1,1-Dioxido-thiomorpholinogruppe und
R eine Gruppe der Formel

$-X-R_5$ oder

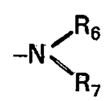 bedeuten, wobei

X ein Sauerstoff- oder Schwefelatom,
$R_5$ eine gegebenenfalls durch eine Phenylgruppe oder eine in 2-, 3- oder 4-Stellung durch eine Hydroxygruppe, eine Dialkylamino- oder Alkoxygruppe mit jeweils 1 bis 3 Kohlenstoffatomen im Alkylteil oder eine Alkoxycarbonylgruppe mit insgesamt 2 bis 4 Kohlenstoffatomen substituierte Alkylgruppe mit 1 bis 4 Kohlenstoffatomen oder, wenn X ein Schwefelatom darstellt, auch eine Phenylgruppe, wobei die vorstehend erwähnten Phenylkerne jeweils durch eine Methylendioxygruppe monosubstituiert oder durch Alkylgruppen mit 1 bis 3 Kohlenstoffatomen, Alkoxygruppen mit 1 bis 3 Kohlenstoffatomen, Alkylthiogruppen mit 1 bis 3 Kohlenstoffatomen, Trifluormethylgruppen und/oder Halogenatome mono- oder disubstituiert sein können, eine Alkylgruppe mit 5 bis 8 Kohlenstoffatomen, eine Cycloalkylgruppe mit 5 bis 7 Kohlenstoffatomen, eine Furfuryl-, Indanyl- oder Naphthylmethylgruppe,

$R_6$ ein Wasserstoffatom, eine Alkylgruppe mit 1 bis 4 Kohlenstoffatomen oder eine durch eine Hydroxygruppe oder Alkoxygruppe mit 1 bis 3 Kohlenstoffatomen in 2-, 3- oder 4-Stellung substituierte Alkylgruppe mit 2 bis 4 Kohlenstoffatomen und

$R_7$ ein Wasserstoffatom, eine gegebenenfalls durch eine Phenylgruppe substituierte Alkylgruppe mit 1 bis 4 Kohlenstoffatomen oder eine Phenylgruppe, wobei die vorstehend erwähnten Phenylkerne jeweils durch eine Methylendioxygruppe monosubstituiert oder durch Alkylgruppen mit 1 bis 3 Kohlenstoffatomen, Alkoxygruppen mit 1 bis 3 Kohlenstoffatomen, Alkylthiogruppen mit 1 bis 3 Kohlenstoffatomen, Trifluormethylgruppen und/oder Halogenatome mono- oder disubstituiert sein können, eine Alkylgruppe mit 5 bis 8 Kohlenstoffatomen, eine Cycloalkylgruppe mit 5 bis 7 Kohlenstoffatomen, eine ab Kohlenstoffatom 2 durch eine Hydroxy- oder Alkoxygruppe mit 1 bis 3 Kohlenstoffatomen substituierte Alkylgruppe mit 2 bis 8 Kohlenstoffatomen, eine Cyclohexylmethyl-, Furfuryl- oder Pyridylmethylgruppe oder

$R_6$ und $R_7$ zusammen mit dem dazwischenliegenden Stickstoffatom eine gegebenenfalls durch eine oder zwei Alkylgruppen mit jeweils 1 bis 3 Kohlenstoffatomen substituierte Alkyleniminogruppe mit 4 bis 7 Kohlenstoffatomen oder eine Tetrahydropyridinogruppe bedeuten, und deren Säureadditionssalze, insbesondere deren physiologisch verträgliche Säureadditionssalze, mit anorganischen oder organischen Säuren.

2. Verbindungen der allgemeinen Formel (I) gemäss Anspruch 1, in der

$R_1$ eine gegebenenfalls ab Kohlenstoffatom 2 mit bis zu 5 Hydroxygruppen substituierte Alkylgruppe mit 2 bis 8 Kohlenstoffatomen, eine in 2-Stellung durch eine Methoxy-, Amino-, Diäthylamino- oder Morpholinogruppe substituierte Äthylgruppe, eine Methyl- oder eine Pyridylmethylgruppe,

$R_2$ ein Wasserstoffatom oder ein gegebenenfalls in 2- oder 3-Stellung durch eine Hydroxy- oder Methoxygruppe substituierte Alkylgruppe mit 1 bis 3 Kohlenstoffatomen,

$R_1$ und $R_2$ zusammen mit dem dazwischenliegenden Stickstoffatom eine gegebenenfalls durch eine Hydroxy-, Methoxy-, Hydroxymethyl-, Dimethylamino-, Acetamino-, Aminocarbonyl- oder Äthoxycarbonylgruppe substituierte Piperidinogruppe, eine Morpholino-, 1-Oxido-thiomorpholino- oder Imidazolylgruppe,

$R_3$ eine Morpholino-, Dimethylmorpholino-, Thiomorpholino-, 1-Oxidothiomorpholino- oder 1,1-Dioxidothiomorpholinogruppe und

R eine gegebenenfalls durch eine Phenylgruppe substituierte Alkoxygruppe mit 1 bis 4 Kohlenstoffatomen, eine Alkoxygruppe mit 5 bis 8 Kohlenstoffatomen oder eine Cyclohexyloxygruppe, eine gegebenenfalls durch eine Phenylgruppe substituierte Alkylthiogruppe mit 1 bis 4 Kohlenstoffatomen oder eine Phenylthiogruppe, wobei die Phenylkerne der vorstehend erwähnten Benzyloxy-, Benzylthio- oder Phenylthiogruppe durch eine Methylendioxygruppe monosubstituiert oder durch Methyl-, Hydroxy-, Methoxy-, Methylthio- oder Trifluormethylgruppen, Fluor-, Chlor- und/oder Bromatome mono- oder disubstituiert sein können, eine Alkylthiogruppe mit 4 bis 8 Kohlenstoffatomen, eine Cyclohexylthio-, 2-Hydroxyäthylthio-, 2-Diäthylaminoäthylthio-, Äthoxycarbonylmethylthio-, α-Methyl-methylthiobenzylthio, Naphthylmethylthio-, Furfurylthio- oder Indanylthiogruppe oder eine Gruppe der Formel

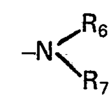 bedeuten, wobei

$R_6$ ein Wasserstoffatom, eine Alkylgruppe mit 1 bis 4 Kohlenstoffatomen oder eine in 2- oder 3-Stellung durch eine Methoxygruppe substituierte Alkylgruppe mit 2 oder 3 Kohlenstoffatomen und

$R_7$ ein Wasserstoffatom, eine gegebenenfalls durch eine Hydroxygruppe substituierte Alkylgruppe mit 2 bis 8 Kohlenstoffatomen, eine in 2- oder 3-Stellung durch eine Methoxy- oder Äthoxygruppe substituierte Alkylgruppe mit 2 oder 3 Kohlenstoffatomen, eine gegebenenfalls durch eine oder 2 Methoxygruppen substituierte Phenylalkylgruppe mit 2 Kohlenstoffatomen im Alkylteil, eine Methyl-, Cyclohexyl-, Cyclohexylmethyl-, 3-Phenylpropyl-, 4-Phenylbutyl-, Furfuryl-, Pyridylmethyl-, Phenyl- oder Benzylgruppe, wobei der Phenylkern der vorstehend erwähnten Phenyl- oder Benzylgruppe durch eine Methylendioxygruppe monosubstituiert oder durch Methyl-, Hydroxy-, Methoxy-, Äthoxy-, Methylthio- oder Trifluormethylgruppen, Fluor- und/oder Chloratome mono- oder disubstituiert sein können, oder

$R_6$ und $R_7$ zusammen mit dem dazwischenliegenden Stickstoffatom eine Pyrrolidino-, Piperidino-, Methylpiperidino-, Äthylpiperidino-, Dimethylpiperidino-, Hexamethylenimino-, Heptamethylenimino- oder Tetrahydropyridinogruppe darstellen, und deren Säureadditionssalze, insbesondere deren physiologisch verträgliche Säureadditionssalze mit anorganischen oder organischen Säuren.

3. Verbindungen der allgemeinen Formel (I) gemäss Anspruch 1, in der

$R_1$, $R_2$ und R wie im Anspruch 2 definiert sind und

$R_3$ die 1-Oxido-thiomorpholinogruppe darstellt, und deren Säureadditionssalze, insbesondere deren physiologisch verträgliche Säureadditionssalze mit anorganischen oder organischen Säuren.

4. Verbindungen der allgemeinen Formel (I) gemäss Anspruch 1, in der

$R_1$ eine ab Kohlenstoffatom 2 gegebenenfalls durch eine oder zwei Hydroxygruppen oder durch eine Methoxygruppe substituierte Alkylgruppe mit 2 bis 5 Kohlenstoffatomen oder eine Methylgruppe,

$R_2$ ein Wasserstoffatom oder eine 2-Hydroxyäthylgruppe,

$R_3$ eine 1-Oxido-thiomorpholinogruppe und

R eine gegebenenfalls durch eine Phenylgruppe substituierte Alkoxy- oder Alkylthiogruppe mit jeweils 1 bis 3 Kohlenstoffatomen, eine Alkoxy- oder Alkylthiogruppe mit jeweils 4 bis 6 Kohlenstoffatomen, eine Cyclohexylthiogruppe oder eine Gruppe der Formel

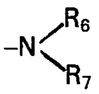 bedeuten, wobei

$R_6$ ein Wasserstoffatom oder eine Alkylgruppe mit 1 bis 4 Kohlenstoffatomen und

$R_7$ eine gegebenenfalls durch eine Hydroxygruppe substituierte Alkylgruppe mit 2 bis 5 Kohlenstoffatomen oder eine durch eine Phenylgruppe substituierte Alkylgruppe mit 1 bis 4 Kohlenstoffatomen, wobei der Phenylkern der oben erwähnten Benzylamino-, Benzyloxy- und Benzylthiogruppe jeweils durch Fluoratome, Chloratome und/oder Methoxygruppen mono- oder disubstituiert sein kann, oder $R_6$ und $R_7$ zusammen mit dem dazwischenliegenden Stickstoffatom eine Pyrrolidino-, Piperidino- oder Hexamethyleniminogruppe darstellen und deren physiologisch verträgliche Säureadditionssalze mit anorganischen oder organischen Säuren.

5. 8-Benzylthio-2- (2-hydroxyäthylamino) -4- (1-oxido-thiomorpholino)- pyrimido[5,4-d]pyrimidin und dessen Säureadditionssalze.

6. 2-(2-Hydroxyäthylamino) -4- (1-oxido-thiomorpholino)-8-(L-1-phenyläthylamino)-pyrimido[5,4-d]pyrimidin und dessen Säureadditionssalze.

7. 8-Benzylamino-2- (2-hydroxyäthylamino) -4- (1-oxido-thiomorpholino)- pyrimido [5,4-d] pyrimidin und dessen Säureadditionssalz.

8. Arzneimittel, enthaltend eine Verbindung der Formel (I) gemäss den Ansprüchen 1 bis 7 oder ein physiologisch verträgliches Säureadditionssalz davon neben einem oder mehreren inerten Trägerstoffen und/oder Verdünnungsmitteln.

9. Verfahren zur Herstellung eines Azrneimittels zur Prophylaxe thrombo-embolischer Erkrankungen, zur Prophylaxe der Arteriosklerose und der Metastasenbildung, dadurch gekennzeichnet, dass eine Verbindung der allgemeinen Formel I gemäss den Ansprüchen 1 bis 7 oder ein physiologisch verträgliches Säureadditionssalz davon in einen oder mehreren inerten Trägerstoffen und/oder Verdünnungsmitteln eingearbeitet wird.

10. Verfahren zur Herstellung von Verbindungen der allgemeinen Formel I gemäss den Ansprüchen 1 bis 7, sowie von deren Säureadditionssalzen, insbesondere von deren physiologisch verträglichen Säureadditionssalzen mit anorganischen oder organischen Säuren, dadurch gekennzeichnet, dass

a) ein Pyrimido[5,4-d]pyrimidin der allgemeinen Formel (II)

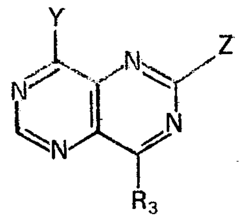 (II)

in der

$R_3$ wie eingangs definiert ist,

Y eine nukleophile Austrittsgruppe darstellt oder die Gruppe R- darstellt, wobei R wie eingangs definiert ist, und

Z eine nukleophile Austrittsgruppe darstellt, mit einem Amin der allgemeinen Formel (III)

H–A III

in der

A eine Gruppe der Formel 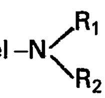 oder 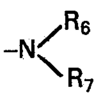

darstellt, wobei $R_1$, $R_2$, $R_6$ und $R_7$ wie eingangs definiert sind, umgesetzt wird oder

b) für den Fall, dass $R = SR_5$ ein Pyrimido [5,4-d]pyrimidin der allgemeinen Formel (IV)

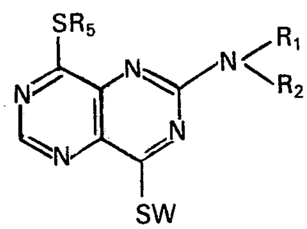 (IV)

in der

$R_1$, $R_2$ und $R_5$ wie eingangs definiert sind und W eine niedere Alkylgruppe oder eine Aralkylgruppe darstellt, mit einem Amin der allgemeinen Formel V

$H–R_3$ V

in der

$R_3$ wie eingangs definiert ist, umgesetzt wird und gewünschtenfalls anschliessend eine erhaltene Verbindung der allgemeinen Formel (I), in der $R_3$ eine gegebenenfalls durch eine oder zwei Alkylgruppen mit jeweils 1 bis 3 Kohlenstoffatomen substituierte Thiomorpholinogruppe darstellt, mittels Oxidation in eine entsprechende 1-Oxido-thiomorpholinoverbindung der allgemeinen Formel (I) übergeführt wird und/oder eine erhaltene Verbindung der allgemeinen Formel (I), in der $R_3$ eine gegebenenfalls durch eine oder zwei Alkylgruppen mit jeweils 1 bis 3 Kohlen-

stoffatomen substituierte Thiomorpholino-oder 1-Oxidothiomorpholinogruppe darstellt, mittels Oxidation in eine entsprechende 1,1-Dioxido-thiomorpholinoverbindung übergeführt wird und/oder eine erhaltene Verbindung der allgemeinen Formel (I) in ihr Säureadditionssalz, insbesondere in ihr physiologisch verträgliches Säureadditionssalz mit einer anorganischen oder organischen Säure, übergeführt wird.

## Revendications

1. Pyrimido[5,4-d]pyrimidines trisubstituées de formule générale (I)

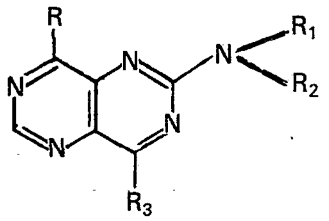

(I)

dans laquelle

$R_1$ représente un groupe alcoyle rectiligne ou ramifié avec 2 à 8 atomes de carbone, éventuellement substitué à partir de l'atome de carbone 2 par jusqu'à 5 groupes hydroxy, ou représente un groupe alcoyle avec 2 à 4 atomes de carbone substitué en position 2-, 3- ou 4- par un groupe alcoxy, amino, alcoylamino, dialcoylamino ou morpholino, les substituants alcoyle mentionnés plus haut pouvant dans chaque cas contenir 1 à 3 atomes de carbone, ou représente un groupe méthyle ou pyridylméthyle,

$R_2$ représente un atome d'hydrogène, un groupe alcoyle avec 1 à 3 atomes de carbone ou un groupe alcoyle avec 2 ou 3 atomes de carbone substitué en position 2- ou 3- par un groupe hydroxy ou un groupe alcoxy avec 1 à 3 atomes de carbone ou

$R_1$ et $R_2$ représentent ensemble avec l'atome d'zote intermédiaire un groupe imidazolyle, morpholino, tiomorpholino, 1-oxydothiomorpholino ou pipéridino, le groupe pipéridino pouvant être substitué par un groupe hydroxy, un groupe alcoxy avec 1 à 3 atomes de carbone, un groupe hydroxyalcoyle avec 1 à 3 atomes de carbone, un groupe alcoxycarbonyle avec en tout 2 à 4 atomes de carbone, un groupe amino, un groupe alcanoylamino avec 1 à 3 atomes de carbone dans la partie alcanoyle, un groupe aminocarbonyle, alcoylamino ou dialcoyalmino avec à chaque fois 1 à 3 atomes de carbone dans la partie alcoyle,

$R_3$ représente un groupe morpholino, thiomorpholino, 1-oxydothiomorpholino ou 1,1-dioxydo-thiomorpholino éventuellement substitué par un ou deux groupes alcoyle avec chacun 1 à 3 atomes de carbone et

R représente un groupe de formule

$-X-R_5$ ou

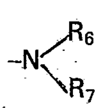

dans lesquels

X représente un atome d'oxygène ou de soufre,

$R_5$ représente un groupe alcoyle avec 1 à 4 atomes de carbone éventuellement substitué par un groupe phényle ou substitué en position 2-, 3- ou 4- par un groupe hydroxy, un groupe dialcoylamino ou alcoxy avec chacun 1 à 3 atomes de carbone dans la partie alcoyle ou un groupe alcoxycarbonyle avec en tout 2 à 4 atomes de carbone ou représente, lorsque X preésente un atome de soufre, également un groupe phényle, les noyaux phényle mentionnés plus haut pouvant chacun être monosubstitué par un groupe méthylènedioxy ou mono- ou disubstitués par des groupes alcoyle avec 1 à 3 atomes de carbone, des groupes alcoxy avec 1 à 3 atomes de carbone, des groupes alcoylthio avec 1 à 3 atomes de carbone, des groupes trifluorométhyle et/ou des atomes d'halogène, ou représente un groupe alcoyle avec 5 à 8 atomes de carbone, un groupe cycloalcoyle avec 5 à 7 atomes de carbone, un groupe furfuryle, indanyle ou naphthylméthyle,

$R_6$ représente un atome d'hydrogène, un groupe alcoyle avec 1 à 4 atomes de carbone ou un groupe alcoyle avec 2 à 4 atomes de carbone substitué en position 2-, 3- ou 4- par un groupe hydroxy ou par un groupe alcoxy avec 1 à 3 atomes de carbone et

$R_7$ représente un atome d'hydrogène, un groupe alcoyle avec 1 à 4 atomes de carbone éventuellement substitué par un groupe phényle ou représente un groupe phényle, les noyaux phényle mentionnés plus haut pouvant chacun être monosubstitué par un groupe méthylènedioxy ou mono- ou disubstitués par des groupes alcoyle avec 1 à 3 atomes de carbone, des groupes alcoxy avec 1 à 3 atomes de carbone, des groupes alcoylthio avec 1 à 3 atomes de carbone, des groupes trifluorométhyle et/ou des atomes d'halogène, ou représente un groupe alcoyle avec 5 à 8 atomes de carbone, un groupe cycloalcoyle avec 5 à 7 atomes de carbone, un groupe alcoyle avec 2 à 8 atomes de carbone substitué à partir de l'atome de carbone 2 par un groupe hydroxy ou alcoxy avec 1 à 3 atomes de carbone, ou représente un groupe cyclohexylméthyle, furfuryle ou pyridylméthyle ou

$R_6$ et $R_7$, ensemble avec l'atome d'azote intermédiaire, représentent un groupe alcoylèneimino avec 4 à 7 atomes de carbone éventuellement substitué par un ou deux groupes alcoyle avec chacun 1 à 3 atomes de carbone ou représentent un groupe tétrahydropyridino, et leurs sels d'addition d'acides, en particulier leurs sels d'addition d'acides physiologiquement supportables, avec des acides minéraux ou organiques.

2. Composés de formule générale (I) selon la revendication 1, dans laquelle

$R_1$ représente un groupe alcoyle avec 2 à 8 atomes de carbone éventuellement substitué à partir de l'atome de carbone 2 par jusqu'à 5 groupes hydroxy, ou représente un groupe éthyle substitué en position 2- par un groupe méthoxy, amino, diéthylamino ou morpholino, ou représente un groupe méthyle ou pyridylméthyle,

$R_2$ représente un atome d'hydrogène ou un

groupe alcoyle avec 1 à 3 atomes de carbone éventuellement substitué en position 2- ou 3- par un groupe hydroxy ou méthoxy,

$R_1$ et $R_2$, ensemble avec l'atome d'azote intermédiaire, représentent un groupe pipéridino éventuellement substitué par un groupe hydroxy, méthoxy, hydroxyméthyle, diméthylamino, acétamino, aminocarbonyle ou éthoxycarbonyle, ou représentent un groupe morpholino, 1-oxydothiomorpholino ou imadazolyle,

$R_3$ représente un groupe morpholino, diméthylmorpholino, thiomorpholino, 1-oxydothiomorpholino ou 1,1-dioxydothiomorpholino et

R représente un groupe alcoxy avec 1 à 4 atomes de carbone éventuellement substitué par un groupe phényle, ou représente un groupe alcoxy avec 5 à 8 atomes de carbone ou un groupe cyclohexyloxy, ou représente un groupe alcoylthio avec 1 à 4 atomes de carbone éventuellement substitué par un groupe phényle ou représente un groupe phénylthio, les noyaux phényle des groupes benzyloxy, benzylthio ou phénylthio mentionnés plus haut pouvant être monosubstitués par un groupe méthylènedioxy ou mono- ou disubstitués par les groupes méthyle, hydroxy, méthoxy, méthylthio ou trifluorométhyle, des atomes de fluor, de chlore et/ou de brome, ou représente un groupe alcoylthio avec 4 à 8 atomes de carbone, un groupe cyclohexylthio, 2-hydroxyéthylthio, 2-diéthylaminoéthylthio, éthoxycarbonylméthylthio, α-méthyl-méthylthiobenzylthio, naphthylméthylthio, furfurylthio ou indanylthio ou un groupe de formule

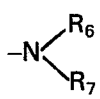

dans laquelle

$R_6$ représente un atome d'hydrogène, un groupe alcoyle avec 1 à 4 atomes de carbone ou un groupe alcoyle avec 2 ou 3 atomes de carbone substitué en position 2- ou 3- par un groupe méthoxy et

$R_7$ représente un atome d'hydrogène, un groupe alcoyle avec 2 à 8 atomes de carbone éventuellement substitué par un groupe hydroxy, ou représente un groupe alcoyle avec 2 ou 3 atomes de carbone substitué en position 2- ou 3- par un groupe méthoxy ou éthoxy, ou représente un groupe phénylalcoyle avec 2 atomes de carbone dans la partie alcoyle, éventuellement substitué par un ou 2 groupes méthoxy ou représente un groupe méthyle, cyclohexyle, cyclohexylméthyle, 3-phénylpropyle, 4-phénylbutyle, furfuryle, pyridylméthyle, phényle ou benzyle, le noyau phényle du groupe phényle ou benzyle mentionné plus haut pouvant être monosubstitué par un groupe méthylènedioxy ou mono- ou disubstitué par des groupes méthyle, hydroxy, méthoxy, éthoxy, méthylthio ou trifluorométhyle, des atomes de fluor et/ou de chlore, ou

$R_6$ et $R_7$ représentent ensemble avec l'atome d'azote intermédiaire un groupe pyrrolidino, pipéridino, méthylpipéridino, éthylpipéridino, diméthylpipéridino, hexaméthyllène-imino, heptaméthylèneimino ou tétrahydropyridino, et leurs sels d'addition d'acides, en particulier leurs sels d'addition d'acides physiologiquement supportables avec des acides minéraux ou organiques.

3. Composés de formule générale (I) selon la revendication 1, dans laquelle

$R_1$, $R_2$ et R sont définis comme dans la revendication 2 et

$R_3$ représente le groupe 1-oxydo-thiomorpholino, et leurs sels d'addition d'acides, en particulier leurs sels d'addition d'acides physiologiquement supportables avec des acides minéraux ou organiques.

4. Composés de formule générale (I) selon la revendication 1, dans laquelle

$R_1$ représente un groupe alcoyle avec 2 à 5 atomes de carbone éventuellement substitué par un ou deux groupes hydroxy ou par un groupe méthoxy, ou représente un groupe méthyle,

$R_2$ représente un atome d'hydrogène ou un groupe 2-hydroxyéthyle,

$R_3$ représente un groupe 1-oxydo-thiomorpholino et

R représente un groupe alcoxy ou alcoylthio avec chacun 1 à 3 atomes de carbone, éventuellement substitué par un groupe phényle, ou représente un groupe alcoxy ou alcoylthio avec chacun 4 à 6 atomes de carbone, ou représente un groupe cyclohexylthio ou un groupe de formule

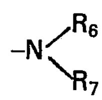

dans laquelle

$R_6$ représente un atome d'hydrogéne ou un groupe alcoyle avec 1 à 4 atomes de carbone et

$R_7$ représente un groupe alcoyle avec 2 à 5 atomes de carbone éventuellement substitué par un groupe hydroxy ou représente un groupe alcoyle avec 1 à 4 atomes de carbone substitué par un groupe phényle, le noyau phényle des groupes benzylamino, benzyloxy et benzylthio mentionnés plus haut pouvant dans chaque cas être mono- ou disubstitué par des atomes de fluor, des atomes de chlore et/ou des groupes méthoxy, ou

$R_6$ et $R_7$ représentent ensemble avec l'atome d'azote intermédiaire un groupe pyrrolidino, pipéridino ou hexaméthylène-imino et leurs sels d'addition d'acides physiologiquement supportables avec des acides minéraux ou organiques.

5. La 8-benzylthio-2- (2-hydroxyéthylamino) -4- (1-oxydo-thiomorpholino)- pyrimido [5,4-d] pyrimidine et ses sels d'addition d'acides.

6. La 2-(2-hydroxyéthylamino) -4- (1-oxydothiomorpholino) -8- (L-1-phényléthylamino) pyrimido[5,4-d]pyrimidine et ses sels d'addition d'acides.

7. La 8-benzylamino-2- (2-hydroxyéthylamino) -4- (1-oxydo-thiomorpholino)-pyrimido [5,4-d] pyrimidine et ses sels d'addition d'acides.

8. Médicament contenant un composé de formule (I) selon les revendications 1 à 7 ou un sel

d'addition d'acide physiologiquement supportable de celui-ci, conjointement à un ou plusieurs excipients et/ou diluants inertes.

9. Procédés pour la préparation d'un médicament pour la prophylaxie des maladies thrombo-embolitiques pour la prophylaxie de l'artériosclérose et de la formation de métastases, caractérisé en ce qu'on introduit un composé de formule générale (I) selon les revendications 1 à 7 ou un sel d'addition d'acide physiologiquement supportable d'un tel composé dans un ou plusieurs excipients et/ou diluants inertes.

10. Procédé pour la préparation de composés de formule générale (I) selon les revendications 1 à 7 ainsi que de leurs sels d'addition d'acides, en particulier de leurs sels d'addition d'acides physiologiquement supportables avec des acides minéraux ou organiques, caractérisé en ce que

a) on fait réagir une pyrimido[5,4-d]pyrimidine de formule générale (II)

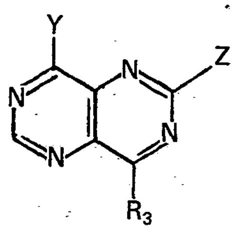

(II)

dans laquelle

$R_3$ est défini comme au début.

Y représente un groupe partant nucléophile ou représente le groupe R–. R étant défini comme au début, et

Z représente un groupe partant nucléophile, avec une amine de formule générale (III)

H–A (III

dans laquelle

A représente un groupe de formule

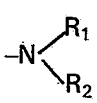 ou 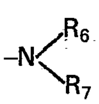

$R_1$, $R_2$, $R_6$ et $R_7$ étant définis comme au début ou

b) dans le cas où $R=SR_5$, on fait réagir une pyrimido[5,4-d]pyrimidine de formule générale (IV)

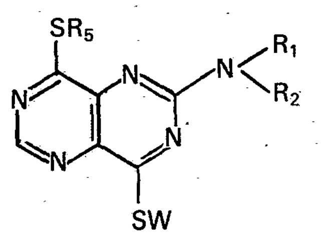

(IV)

dans laquelle

$R_1$ $R_2$ et $R_5$ sont définis comme au début et

W représente un groupe alcoyle inférieur ou un groupe aralcoyle, avec une amine de formule générale V

H–$R_3$ V

dans laquelle

$R_3$ est défini comme au début, et si on le désire ensuite, on transforme un composé obtenus de formule générale (I), dans laquelle $R_3$ représente un groupe thiomoropholino éventuellement substitué par un ou deux groupes alcoyle avec chacun 1 à 3 atomes de carbone, au moyen d'une oxydation en un composé 1-oxydo-thiomorpholino correspondant de formule générale (I)

et/ou on transforme un composé obtenu de formule générale (I) dans laquelle $R_3$ représente un groupe thiomorpholino ou 1-oxydothiormopholino éventuellement substitué par un ou deux groupes alcoyle avec chacun 1 à 3 atomes de carbone, ou moyen d'une oxydation en un composé 1,1-dioxydo-thiomorpholino correspondant

et/ou on transforme un composé obtenu de formule générale (I) en son sel d'addition d'acide, en particulier en son sel d'addition d'acide physiologiquement supportable avec des acides minéraux ou organiques.

## Claims

1. Trisubstituted pyrimido [5,4–d] pyrimidines of general formula

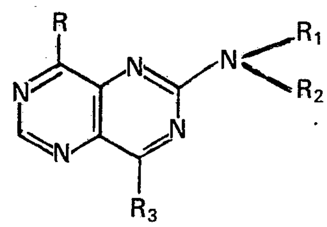

(I)

in which

$R_1$ represents a straight-chained or branched alkyl group with 2 to 8 carbon atoms optionally substituted from carbon atom 2 with up to 5 hydroxy groups, an alkyl group with 2 to 4 carbon atoms substituted in 2-, 3- or 4-position by an alkoxy, amino, alkylamino, dialkylamino or morpholino group, whilst the above-mentioned alkyl substituents may each contain 1 to 3 carbon atoms, a methyl or pyridylmethyl group,

$R_2$ represents a hydrogen atom, an alkyl group with 1 to 3 carbon atoms or an alkyl group with 2 or 3 carbon atoms substituted in 2- or 3-position by a hydroxy group or an alkoxy group with 1 to 3 carbon atoms, or

$R_1$ and $R_2$ represent, together with the nitrogen atom between them, an imidazolyl, morpholino, thiomorpholino, 1-oxido-thiomorpholino or piperidino group, whilst the piperidino group can be substituted by a hydroxy group, an alkoxy group with 1 to 3 carbon atoms, a hydroxyalkyl group with 1 to 3 carbon atoms, an alkoxycarbonyl group with 2 to 4 carbon atoms altogether, an amino group, an alkanoylamino group with 1 to 3 carbon atoms in the alkanoyl part, an aminocarbonyl, alkylamino or dialkylamino group, each with 1 to 3 carbon atoms in the alkyl part,

$R_3$ represents a morpholino, thiomorpholino, 1-oxidothiomorpholino or 1,1-dioxidothiomorpholino group optionally substituted by one or two alkyl groups, each with 1 to 3 carbon atoms, and

R represents a group of formula

$-X-R_5$ or

$-N\begin{smallmatrix}R_6\\R_7\end{smallmatrix}$ in which

X represents an oxygen or sulphur atom,

$R_5$ represents an alkyl group with 1 to 4 carbon atoms optionally substituted by a phenyl group or an alkoxycarbonyl group with 2 to 4 carbon atoms altogether or in 2-, 3- or 4-position by a hydroxy group, a dialkylamino or alkoxy group each with 1 to 3 carbon atoms in the alkyl part,

or, if X represents a sulphur atom, also a phenyl group, whilst the above-mentioned phenyl nuclei can each be monosubstituted by a methylenedioxy group or mono- or disubstituted by alkyl groups with 1 to 3 carbon atoms, alkoxy groups with 1 to 3 carbon atoms, alkylthio groups with 1 to 3 carbon atoms, trifluoromethyl groups and/or halogen atoms, an alkyl group with 5 to 8 carbon atoms, a cycloalkyl group with 5 to 7 carbon atoms, a furfuryl, indanyl or naphthylmethyl group,

$R_6$ represents a hydrogen atom, an alkyl group with 1 to 4 carbon atoms or an alkyl group with 2 to 4 carbon atoms substituted in 2-, 3- or 4-position by a hydroxy group or alkoxy group with 1 to 3 carbon atoms, and

$R_7$ represents a hydrogen atom, an alkyl group with 1 to 4 carbon atoms optionally substituted by a phenyl group, or a phenyl group, whilst the above-mentioned phenyl nuclei can each be monosubstituted by a methylenedioxy group or mono- or disubstituted by alkyl groups with 1 to 3 carbon atoms, alkoxy groups with 1 to 3 carbon atoms, alkylthio groups with 1 to 3 carbon atoms, trifluoromethyl groups and/or halogen atoms, an alkyl group with 5 to 8 carbon atoms, a cycloalkyl group with 5 to 7 carbon atoms, an alkyl group with 2 to 8 carbon atoms substituted from carbon atom 2 by a hydroxy or alkoxy group, each with 1 to 3 carbon atoms, a cyclohexylmethyl, furfuryl or pyridylmethyl group, or

$R_6$ and $R_7$ represent, together with the nitrogen atom between them, an alkyleneimino group with 4 to 7 carbon atoms optionally substituted by one or two alkyl groups, each with 1 to 3 carbon atoms or a tetrahydropyridino group, and their acid addition salts, especially their physiologically compatible acid addition salts, with inorganic or organic acids.

2. Compounds of general formula I according to claim 1, in which

$R_1$ represents an alkyl group with 2 to 8 carbon atoms optionally substituted from carbon atom 1 with up to 5 hydroxy groups, an ethyl group substituted in 2-position by a methoxy, amino, diethylamino or morpholino group, a methyl or a pyridylmethyl group,

$R_2$ represents a hydrogen atom or an alkyl group with 1 to 3 carbon atoms optionally substituted in 2- or 3-position by a hydroxy or methoxy group,

$R_1$ and $R_2$ represent, together with the nitrogen atom between them, a piperidino group optionally substituted by a hydroxy, methoxy, hydroxymethyl, dimethylamino, acetamino, aminocarbonyl or ethoxycarbonyl group, a morpholino, 1-oxidothiomorpholino or imidazolyl group,

$R_3$ represents a morpholino, dimethylmorpholino, thiomorpholino, 1-oxidothiomorpholino or 1,1-dioxidothiomorpholino group, and

R represents an alkoxy group with 1 to 4 carbon atoms optionally substituted by a phenyl group, an alkoxy group with 5 to 8 carbon atoms or a cyclohexyloxy group, an alkylthio group with 1 to 4 carbon atoms optionally substituted by a phenyl group, or a phenylthio group, whilst the phenyl nuclei of the above-mentioned benzyloxy, benzylthio or phenylthio group can be monosubstituted by a methylendioxy group or mono- or disubstituted by methyl, hydroxy, methoxy, methylthio or trifluoromethyl groups, fluorine, chlorine and/or bromine atoms, an alkylthio group with 4 to 8 carbon atoms, a cyclohexylthio, 2-hydroxyethylthio, 2-diethylaminoethylthio, ethoxycarbonylmethylthio, α-methyl-methylthiobenzylthio, naphthylmethylthio, furfurylthio or indanylthio group or a group of formula

$-N\begin{smallmatrix}R_6\\R_7\end{smallmatrix}$ in which

$R_6$ represents a hydrogen atom, an alkyl group with 1 to 4 carbon atoms or an alkyl group with 2 or 3 carbon atoms substituted in 2- or 3-position by a methoxy group, and

$R_7$ represents a hydrogen atom, an alkyl group with 2 to 8 carbon atoms optionally substituted by a hydroxy group, an alkyl group with 2 or 3 carbon atoms substituted in 2- or 3-position by a methoxy or ethoxy group, a phenylalkyl group with 2 carbon atoms in the alkyl part, optionally substituted by one or two methoxy groups, a methyl, cyclohexyl, cyclohexylmethyl, 3-phenylpropyl, 4-phenylbutyl, furfuryl, pyridylmethyl, phenyl or benzyl group, whilst the phenyl nucleus of the above-mentioned phenyl or benzyl group can be monosubstituted by a methylenedioxy group or mono- or disubstituted by methyl, hydroxy, methoxy, ethoxy, methylthio or trifluoromethyl groups, fluorine and/or chlorine atoms, or

$R_6$ and $R_7$ represent, together with the nitrogen atom between them, a pyrrolidino, piperidino, methylpiperidino, ethylpiperidino, dimethylpiperidino, hexamethyleneimino, heptamethyleneimino or tetrahydropyridino group, and their acid addition salts, especially their physiologically compatible acid addition salts with inorganic or organic acids.

3. Compounds of general formula I according to claim 1, in which

$R_1$, $R_2$ and R are as defined in claim 2, and $R_3$ represents the 1-oxidothiomorpholino group, and their acid addition salts, especially their physiologically compatible acid addition salts with inorganic or organic acids.

4. Compounds of general formula I according to claim 1, in which

$R_1$ represents an alkyl group with 2 to 5 carbon atoms optionally substituted from carbon atom 2 by one or two hydroxy groups or by a methoxy group, or a methyl group,

$R_2$ represents a hydrogen atom or a 2-hydroxyethyl group,

$R_3$ represents a 1-oxidothiomorpholino group, and

R represents an alkoxy or alkylthio group, each with 1 to 3 carbon atoms, optinally substituted by a phenyl group, an alkoxy or alkylthio group, each with 4 to 6 carbon atoms, a cyclohexylthio group or a group of formula

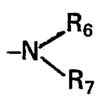 in which

$R_6$ represents a hydogen atom or an alkyl group with 1 to 4 carbon atoms, and

$R_7$ represents an alkyl group with 2 to 5 carbon atoms optionally substituted by a hydroxy group, or an alkyl group with 1 to 4 carbon atoms substituted by a phenyl group, whilst the phenyl nucleus of the above-mentioned benzylamino, benzyloxy and benzylthio group can be mono- or disubstituted in each case by fluorine or chlorine atoms and/or by methoxy groups, or

$R_6$ and $R_7$ represent, together with the nitrogen atom between them, a pyrrolidino, piperidino or hexamethyleneimino group, and their physiologically compatible acid addition salts with inorganic or organic acids.

5. 8-Benzylthio-2-(2-hydroxyethylamino)-4-(1-oxidothiomorpholino)-pyrimido[5,4-d]pyrimidine and its acid addition salts.

6. 2-(2-Hydroxyethylamino)-4-(1-oxidothiomorpholino)-8-(L-1-phenylethylamino)-pyrimido[5,4-d]pyrimidine and its acid addition salts.

7. 8-Benzylamino-2-(2-hydroxyethylamino)-4-(1-oxidothiomorpholino)-pyrimido[5,4-d]pyrimidine and its acid addition salts.

8. Pharmaceuticals containing a compound of formula I according to claims 1 to 7 or a physiologically acceptable acid addition salt thereof in addition to one or more inert carriers and/or diluents.

9. Process for preparing a pharmaceutical composition for the prophylaxis of thromboembolic diseases and arteriosclerosis and metastasis formation, characterised in that a compound of general formula I as claimed in claims 1 to 7 or a physiologically acceptable acid addition salt thereof is incorporated in one or more inert carriers and/or diluents.

10. Process for the preparation of compounds of general formula I as claimed in claims 1 to 7, and of their acid addition salts, especially their physiologically compatible acid addition salts with inorganic or organic acids, characterised in that

a) a pyrimido[5,4-d]pyrimidine of general formula (II)

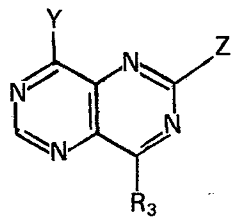 (II)

in which

$R_3$ is as hereinbefore defined,

Y represents a nucleophilic leaving group or the group R-, where R is as defined hereinbefore, and

Z represents a nucleophilic leaving group, is reacted with an amine of general formula (III)

H–A (III)

in which

A represents a group of formula

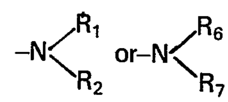

or

where $R_1$, $R_2$, $R_6$ ans $R_7$ are as defined hereinbefore, or

b) if R = $SR_5$ a pyrimido[5,4-d]pyrimidine of general formula (IV)

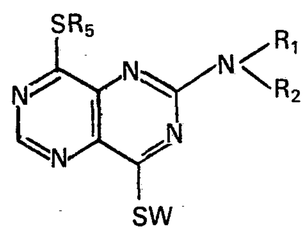 (IV)

in which

$R_1$, $R_2$ and $R_5$ are as defined hereinbefore and W represents a lower alkyl group or an aralkyl group, is reacted with an amine of general formula (V)

$H–R_3$ (V)

in which

$R_3$ is as defined hereinbefore, and, if desired, a compound of general formula I obtained, in which $R_3$ represents a thiomorpholino group optionally substituted by one or two groups, each with 1 to 3 carbon atoms, is subsequently converted by means of oxidation into a corresponding 1-oxidothiomorpholino compound of general formula I, and/or a compound of general formula I obtained, in which $R_3$ represents a thiomorpho-

lino or 1-oxidothiomorpholino group optionally substituted by one or two alkyl groups, each with 1 to 3 carbon atoms, is converted by means of oxidation into a corresponding 1,1-dioxidothiomorpholino compound, and/or a compound of general formula I obtained is converted into its acid addition salt, especially into its physiologically compatible acid addition salt with inorganic or organic acid.